# EUROPEAN PATENT APPLICATION

(11) **EP 4 112 719 A1**
(43) Date of publication of application: **04.01.2023**
(21) Application number: 20848686.0
(22) Date of filing: 16.10.2020
(51) Int. Cl.: C12N 5/10, C12N 1/15, C12N 1/19, C12N 1/21, C12N 15/09

(54) **GENE KNOCK-IN METHOD, METHOD FOR PRODUCING GENE KNOCK-IN CELL, GENE KNOCK-IN CELL, CANCERATION RISK EVALUATION METHOD, CANCER CELL PRODUCTION METHOD, AND KIT FOR USE IN SAME**

(30) Priority: 26.02.2020 JP 2020030740; 12.06.2020 JP 2020102122
(71) Applicant: AISIN CORPORATION, Kariya-shi, Aichi 448-8650 (JP)
(72) Inventor: KATAYAMA, Shota, Sapporo-shi, Hokkaido 004-0015 (JP)
(74) Representative: TBK
(86) International application number: PCT/JP2020/039169
(87) International publication number: WO 2021/171688

(57) **Abstract**

A gene knock-in method for knocking in an exogenous DNA into a target site of a genomic DNA, the method comprising:
a step of introducing a donor DNA that contains the exogenous DNA and a CRISPR-Cas9 system that includes a Cas9 protein and a guide RNA for the Cas9 protein as constituent elements into a cell, wherein
the donor DNA contains in order from a 5' side: a 5'-microhomology region formed of a first' nucleotide sequence that is capable of being joined to a first nucleotide sequence on a 5' side of the target site of the genomic DNA by microhomology-mediated end joining; the exogenous DNA; and a 3'-microhomology region formed of a second' nucleotide sequence that is capable of being joined to a second nucleotide sequence on a 3' side of the target site of the genomic DNA by microhomology-mediated end joining,
the CRISPR-Cas9 system
cleaves, in the genomic DNA, a first cleavage target region between the target site and the first nucleotide sequence and a second cleavage target region between the target site and the second nucleotide sequence, and
cleaves, in the donor DNA, a third cleavage target region on the 5' side of the 5'-microhomology region, a fourth cleavage target region between the exogenous DNA and the 5'-microhomology region, a fifth cleavage target region between the exogenous DNA and the 3'-microhomology region, and a sixth cleavage target region on the 3' side of the 3'-microhomology region, and

the exogenous DNA is inserted between the first nucleotide sequence and the second nucleotide sequence of the genomic DNA.

## Description

### [Technical Field]

The present invention relates to a gene knock-in method, a gene knock-in cell fabrication method, a gene knock-in cell, a malignant transformation risk evaluation method, a cancer cell production method, and a kit for use in these.

### [Background Art]

Since the CRISPR-Cas9 system (clustered regularly interspaced short palindromic repeats/CRISPR associated proteins 9) was reported to be available as a restriction enzyme in 2012, the CRISPR-Cas9 system has been revealed to function in various biological species and has become a versatile gene modification tool utilized by researchers all over the world. DNA editing techniques using the CRISPR-Cas9 system typically cleave the target DNA with the CRISPR-Cas9 system and utilize the repair mechanisms of the cleaved target DNA to be repaired. However, the current techniques are not suitable for DNA editing that requires replacement of long-chain DNA since mutations that these techniques are capable of conducting are deletion and insertion of several bases or so.

In addition, the applications of the CRISPR-Cas9 system for various purposes have been studied so far, and for example, International Publication No. WO2017/090724 A (WO 2017-090724 A) (PTL 1) describes the methylation of a target DNA using a fusion protein of an inactivated CRISPR-associated endonuclease Cas9 (dCas9) having no nuclease activity and a tag peptide array, a fusion protein of a tag peptide binding portion and methyltransferase or the like, and a guide RNA. The methylation of a genomic DNA is useful particularly because it is possible to suppress the expression of a specific gene by methylating the whole or part of a promoter of the gene. However, although there is a report that in order to completely suppress the expression of the gene, it is necessary for 100% of DNA up to 500 bp upstream of of the transcription start site to have been methylated, the region that can be methylated by the method described in PTL 1 is approximately 100 bp, and there is no report on a method that can methylate such a long-chain target DNA.

For example, many sequence abnormalities of genes have been reported as factors that are associated with malignant transformation of a cell. As a factor that is not associated with the sequence abnormality, the methylation of a genomic DNA (that is, cytosine methylation) and accumulation of methylation has been known. However, since it has been difficult to methylate a long-chain genomic DNA in cells as described above, the methylation of a genomic DNA has not been sufficiently studied yet. Note that as of the factor associated with the malignant transformation of a cell, for example, JP 2019-60749 A (PTL 2) describes a method for evaluating a malignant transformation risk by using a set of evaluation elements in the blood serum as one of indicators. However, such an indicator is set based on statistical data calculated from people of a positive group which are diagnosed with cancers and people of a negative group which are not diagnosed with cancers, and conventionally there has been no method for directly evaluating a malignant transformation risk based on factors associated with the above-described malignant transformation.

In addition, as another DNA editing technique that is capable of conducting deletion, insertion, replacement, and the like of a relatively long-chain base on a target DNA, there is a gene targeting (knockout or knock-in) technique that rewrites the sequence of a target DNA by using exogenous DNA. Moreover, methods in which this technique is combined with the CRISPR-Cas9 system have also been studied, and for example, Sakuma et al., Nat protocol, Vol. 11 No. 1,2016, p. 118-133 (NPL 1), Katayama et al., Angew Chem Int Ed, 55(22), 2016, p. 6452-6456 (NPL 2), Katayama et al., Life Sci Alliance, Vol. 3 No. 1, 2019, e201900528 (NPL 3), and the like describe methods for knocking in an exogenous DNA having a relatively long-chain (for example, 700 bp or more) into a target site of a genomic DNA by combining a mechanism that recognizes and repairs a short sequence (5 to 25 bp) in a terminal portion after DNA cleavage, which is called a microhomology (MMEJ: microhomology-mediated end joining) with the CRISPR-Cas9 system. However, it cannot be said that the methods described in these literatures are sufficient in terms of gene knock-in efficiency (for example, approximately 20% in the method described in NPL 3).

### [Citation List]

### [Patent Literatures]

[PTL 1] International Publication No. WO2017/090724 A [PTL 2] Japanese Unexamined Patent Application Publication No. 2019-60749

### [Non Patent Literatures]

[NPL 1] Sakuma et al., Nat protocol, Vol. 11 No. 1, 2016, p. 118-133
[NPL 2] Katayama et al., Angew Chem Int Ed ,55(22), 2016, p. 6452-6456
[NPL 3] Katayama et al., Life Sci Alliance, Vol. 3 No. 1, 2019, e201900528

### [Summary of Invention]

### [Technical Problem]

The present invention has been made in view of the problems of the above-described conventional techniques, and an object of the present invention is to provide a novel gene knock-in method, gene knock-in cell fabrication method, gene knock-in cell, and kit for use in these that are capable of inserting a long-chain exogenous DNA into a target site of a genomic DNA more efficiently than the conventional techniques.

Moreover, the present invention aims to provide a novel malignant transformation risk evaluation method that is capable of directly evaluating malignant transformation risk of cell by methylating a promoter of a genomic DNA of the cell and a cancer cell production method that does not cause sequence abnormality of a gene due to methylation of a promoter of a genomic DNA of the cell.

### [Solution to Problem]

As a result of conducting earnest studies in order to achieve the above-described object, the present inventors found that in a method for knocking in an exogenous DNA into a target site of a genomic DNA, by introducing a donor DNA that contains the exogenous DNA and a CRISPR-Cas9 system that includes a Cas9 protein and a guide RNA for the Cas9 protein as constituent elements into a cell, making the donor DNA contain in order from a 5' side: a first' nucleotide sequence (5'-microhomology region) that is capable of being joined to a first nucleotide sequence on a 5' side of the target site of the genomic DNA by microhomology-mediated end joining; the exogenous DNA; and a second' nucleotide sequence (3'-microhomology region) that is capable of being joined to a second nucleotide sequence on a 3' side of the target site of the genomic DNA by microhomology-mediated end joining, and making the CRISPR-Cas9 system cleave between the target site and the first nucleotide sequence (first cleavage target region) and between the target site and the second nucleotide sequence (second cleavage target region) in the genomic DNA, as well as a 5' side of the 5'-microhomology region (third cleavage target region), between the exogenous DNA and the 5'-microhomology region (fourth cleavage target region), between the exogenous DNA and the 3'-microhomology region (fifth cleavage target region), and a 3' side of the 3'-microhomology region (sixth cleavage target region) in the donor DNA, it is made possible to insert the exogenous DNA having a relatively long-chain(for example, 700 bp or more) between the first nucleotide sequence and the second nucleotide sequence of the genomic DNA more efficiently than the conventional techniques.

Furthermore, the present inventors found that by introducing methylation into a promoter of a genomic DNA in a cell to completely suppress the expression of a gene under control of the promoter and then confirming whether or not the cell turns into center through malignant transformation, it is possible to directly evaluate malignant transformation risk of the cell. In addition, the present inventors also found that this method also makes it possible to evaluate whether a gene under control of the promoter is a gene involved in the malignant transformation by using whether or not the cell turns into cancer as an indicator. Moreover, the present inventors found that by using a cell derived from a test subject as the cell and a promoter of a gene related to a specific cancer as the promoter, it is possible to evaluate malignant transformation risk in the future (risk of development of the specific cancer) directly in the test subject without depending on statistical indicator. In addition, the present inventors found that by introducing methylation into this promoter, it is also possible to produce a cancer cell without causing sequence abnormality of a gene. These findings have led to the completion of the present invention.

Aspects of the present invention obtained by the above-described findings are as follows:
[1] A gene knock-in method for knocking in an exogenous DNA into a target site of a genomic DNA, the method comprising:
   a step of introducing a donor DNA that contains the exogenous DNA and a CRISPR-Cas9 system that includes a Cas9 protein and a guide RNA for the Cas9 protein as constituent elements into a cell, wherein
   the donor DNA contains in order from a 5' side: a 5'-microhomology region formed of a first' nucleotide sequence that is capable of being joined to a first nucleotide sequence on a 5' side of the target site of the genomic DNA by microhomology-mediated end joining; the exogenous DNA; and a 3'-microhomology region formed of a second' nucleotide sequence that is capable of being joined to a second nucleotide sequence on a 3' side of the target site of the genomic DNA by microhomology-mediated end joining,
   the CRISPR-Cas9 system
      cleaves, in the genomic DNA, a first cleavage target region between the target site and the first nucleotide sequence and a second cleavage target region between the target site and the second nucleotide sequence, and
      cleaves, in the donor DNA, a third cleavage target region on the 5' side of the 5'-microhomology region, a fourth cleavage target region between the exogenous DNA and the 5'-microhomology region, a fifth cleavage target region between the exogenous DNA and the 3'-microhomology region, and a sixth cleavage target region on the 3' side of the 3'-microhomology region, and
   the exogenous DNA is inserted between the first nucleotide sequence and the second nucleotide sequence of the genomic DNA.
[2] The gene knock-in method according to [1], wherein
   the first cleavage target region and the second cleavage target region of the genomic DNA as well as the third cleavage target region, the fourth cleavage target region, the fifth cleavage target region, and the sixth cleavage target region of the donor DNA respectively contain a first guide RNA-target sequence, a second guide RNA-target sequence, a third guide RNA-target sequence, a fourth guide RNA-target sequence, a fifth guide RNA-target sequence, and a sixth guide RNA-target sequence that are recognized by the guide RNA.
[3] The gene knock-in method according to [1] or [2], wherein
   the Cas9 protein is a protein that contains point mutations of N692A, M694A, Q695A, and H698A in a wild-type Streptococcus pyogenes Cas9 (SpCas9) protein.
[4] The gene knock-in method according to any one of 1] to [3], wherein
   the exogenous DNA of the donor DNA has 700 bases or more.
[5] The gene knock-in method according to any one of [1] to [4], wherein
   the exogenous DNA of the donor DNA is methylated.
[6] The gene knock-in method according to [5], wherein
   the target site of the genomic DNA is contained in a promoter, and
   the exogenous DNA is the target site that is methylated.
[7] A gene knock-in cell fabrication method for fabricating a cell in which the exogenous DNA is inserted into a target site of a genomic DNA by using the gene knock-in method according to any one of [1] to [6] .
[8] A gene knock-in cell in which an exogenous DNA containing 700 bp or more methylated base pairs is introduced.
[9] A malignant transformation risk evaluation method for evaluating malignant transformation risk of a target cell, the method comprising:
   a methylation step of methylating a target promoter site contained in a promoter of a genomic DNA in the target cell;
   a detection step of detecting malignant transformation of the target cell; and
   a cell evaluation step of evaluating malignant transformation risk of the cell by using the malignant transformation of the target cell as an indicator.
[10] The malignant transformation risk evaluation method according to [9], wherein
   a length of the target promoter site is 700 bp or more.
[11] The malignant transformation risk evaluation method according to [9] or [10], wherein
   the methylation step is a step of knocking in an exogenous methylated promoter site into the target promoter site of a genomic DNA of the target cell by using the gene knock-in method according to any one of [1] to [6],
   the target site is the target promoter site, and
   the exogenous DNA is the methylated promoter site.
[12] The malignant transformation risk evaluation method according to any one of [9] to [11], further comprising:
   a gene evaluation step of evaluating malignant transformation capability of a gene controlled by a promoter containing the target promoter site by using the malignant transformation of the target cell as an indicator.
[13] The malignant transformation risk evaluation method according to any one of [9] to [11], wherein
   the target cell is a cell derived from a test subject, and
   the promoter containing the target promoter site is a promoter of at least one gene selected from the group consisting of SP3, CDKN2A, p53, p21, and TERT1,
   the method further comprising:
      a test subject evaluation step of evaluating malignant transformation risk of the test subject by using the malignant transformation of the target cell as an indicator.
[14] The malignant transformation risk evaluation method according to [13], wherein
   the target cell is at least one selected from the group consisting of a B cell, a liver cell, a gastric epithelial cell, and a mucosal cell of pancreatic duct.
[15] A cancer cell production method comprising:
   a step of obtaining a cancer cell by methylating a target promoter site contained in a promoter of a genomic DNA in a cell.
[16] The cancer cell production method according to [15], wherein
   a length of the target promoter site is 700 bp or more.
[17] The cancer cell production method according to [15] or [16], wherein
   the methylation is methylation to knock in an exogenous methylated promoter site into the target promoter site of a genomic DNA of the cell by using the gene knock-in method according to any one of [1] to [6],
   the target site is the target promoter site, and
   the exogenous DNA is the methylated promoter site.
[18] A gene knock-in kit for use in the gene knock-in method according to any one of [1] to [6], the kit comprising:
   at least one selected from the group consisting of
   (a) a Cas9 protein cleaving a first cleavage target region and a second cleavage target region of the genomic DNA as well as a third cleavage target region, a fourth cleavage target region, a fifth cleavage target region, and a sixth cleavage target region of the donor DNA, a polynucleotide encoding the Cas9 protein, or a vector expressing the Cas9 protein; and
   (b) a guide RNA of the Cas9 protein, a polynucleotide encoding the guide RNA, or a vector expressing the guide RNA.
[19] The gene knock-in kit according to [18], wherein
   the guide RNA contains a first guide RNA, a second guide RNA, a third guide RNA, a fourth guide RNA, a fifth guide RNA, and a sixth guide RNA that respectively recognize a first guide RNA-target sequence and a second guide RNA-target sequence of the genomic DNA as well as a third guide RNA-target sequence, a fourth guide RNA-target sequence, a fifth guide RNA-target sequence, and a sixth guide RNA-target sequence of the donor DNA.
[20] The gene knock-in kit according to [18] or [19], wherein
   the Cas9 protein is a protein containing point mutations of N692A, M694A, Q695A, and H698A in a wild-type Streptococcus pyogenes Cas9 (SpCas9) protein.

### [Advantageous Effects of Invention]

The present invention makes it possible to provide a novel gene knock-in method, gene knock-in cell fabrication method, gene knock-in cell, and kit for use in these that are capable of inserting a long-chain exogenous DNA into a target site of a genomic DNA more efficiently than the conventional techniques.

In addition, since the present invention makes it possible to insert an exogenous DNA having a long-chain (for example, 700 bp or more) and/or an exogenous DNA modified outside the cell into a target site of a genomic DNA highly efficiently, it is possible to suppress the expression of a specific gene by inserting an exogenous DNA having 700 bp or more 100%-methylated base pairs into a promoter of the gene, for example.

Moreover, the present invention makes it possible to provide a novel malignant transformation risk evaluation method that is capable of directly evaluating malignant transformation risk of a cell by methylating a promoter of a genomic DNA of the cell, and a cancer cell production method that does not cause sequence abnormality of a gene due to methylation of a promoter of a genomic DNA of a cell.

Hence, the present invention also makes it possible to provide a method (gene evaluation method) that is capable of evaluating whether or not the gene under control of the promoter is a gene involved in malignant transformation, and in the case where the cell is a cell derived from a test subject and the promoter is a promoter of a gene related to specific cancer, a method (test subject evaluation method) that is capable of evaluating malignant transformation risk in the future (risk of development of the specific cancer) directly in the test subject without depending on statistical indicators.

### [Brief Description of Drawings]

[Fig. 1] Fig. 1 is schematic diagrams showing examples of modes of a target DNA region (a) of a genomic DNA and of a donor DNA (b) according to the present invention.
[Fig. 2] Fig. 2 is a graph showing a relation between the concentration of puromycin and the number of viable cells (relative number of cells) on day 2 from the start of the addition of puromycin.
[Fig. 3] Fig. 3 is a graph showing a relation between the concentration of puromycin and the number of viable cells (relative number of cells) on day 3 from the start of the addition of puromycin.
[Fig. 4] Fig. 4 is an electrophoresis picture after a T7EI treatment.
[Fig. 5] Fig. 5 is electrophoresis pictures of RT-PCR products after gene knock-in.
[Fig. 6] Fig. 6 is a graph showing the proportions of methylated cytosine in hSP3 promoters after the gene knock-in.
[Fig. 7] Fig. 7 is pictures showing external appearances after introduction of methylation and Crystal Violet staining in B cells derived from Person C.
[Fig. 8] Fig. 8 is pictures showing external appearances after introduction of methylation and Crystal Violet staining in B cells derived from Person D.
[Fig. 9] Fig. 9 is pictures showing external appearances after introduction of methylation and Crystal Violet staining in B cells derived from Person E.
[Fig. 10] Fig. 10 is pictures showing external appearances after introduction of methylation and Crystal Violet staining in B cells derived from Person F.

### [Description of Embodiments]

Hereinafter, the present invention will be described in detail in accordance with preferred embodiments.

### <Gene Knock-in Method>

The present invention is a gene knock-in method for knocking in an exogenous DNA to a target site of a genomic DNA, the method comprising:
a step of introducing a donor DNA that contains the exogenous DNA and a CRISPR-Cas9 system that includes a Cas9 protein and a guide RNA for the Cas9 protein as constituent elements into a cell, wherein
the donor DNA contains in order from a 5' side: a 5'-microhomology region formed of a first' nucleotide sequence that is capable of being joined to a first nucleotide sequence on a 5' side of the target site of the genomic DNA by microhomology-mediated end joining; the exogenous DNA; and a 3'-microhomology region formed of a second' nucleotide sequence that is capable of being joined to a second nucleotide sequence on a 3' side of the target site of the genomic DNA by microhomology-mediated end joining,
the CRISPR-Cas9 system
   cleaves, in the genomic DNA, a first cleavage target region between the target site and the first nucleotide sequence and a second cleavage target region between the target site and the second nucleotide sequence, and
   cleaves, in the donor DNA, a third cleavage target region on the 5' side of the 5'-microhomology region, a fourth cleavage target region between the exogenous DNA and the 5'-microhomology region, a fifth cleavage target region between the exogenous DNA and the 3'-microhomology region, and a sixth cleavage target region on the 3' side of the 3'-microhomology region, and
the exogenous DNA is inserted between the first nucleotide sequence and the second nucleotide sequence of the genomic DNA.

It has been reported that in cleavage target portions cleaved by an artificial DNA-cleaving enzyme such as Cas9, microhomology-mediated end joining (MMEJ) in which complementary sequences of the cleaved ends are joined to be repaired is highly likely to occur. With this as the background, in the methods described in NPLs 1 to 3, sequences that are recognized by a guide RNA of the CRISPR-Cas9 are added to both ends of an exogenous DNA in the donor DNA to be introduced into a cell as well, and the Cas9 is caused to cleave three or four portions in total, that is, a cleavage target portion on the genomic DNA (one portion or two portions at both ends of a target site (site to which the exogenous DNA is knocked in)) and two portions at both ends of the exogenous DNA on the donor DNA. Moreover, in the methods described in NPLs 1 to 3, the donor DNA was designed to be joined to the cleaved ends on the genomic DNA at the time of the double-stranded cleavage by microhomology-mediated end joining. In addition, the cleaved ends at both ends of the exogenous DNA, which are generated by the Cas9, are also joined to the cleaved ends on the genomic DNA, which are generated by the Cas9, by the microhomology-mediated end joining, it is possible to highly efficiently insert (knock-in) the exogenous DNA into the cleavage target portion on the genomic DNA.

In contrast, in the gene knock-in method of the present invention, sequences that are recognized by a guide RNA of the CRISPR-Cas9 are further added to two portions outside (5' side and 3' side based on the exogenous DNA) of the sequences (the 5'-microhomology region and the 3'-microhomology region) to be joined by microhomology-mediated end joining in a donor DNA to be introduced into a cell, and the Cas9 is caused to cleave six portions in total, that is, cleavage target portions on the genomic DNA (two portions at both ends of the target site) and four portions including two portions on each of the 5' side and the 3' side of the exogenous DNA on the donor DNA. For this reason, in the state where the exogenous DNA and the target site have aligned, it is possible for at least three Cas9s and a pair of microhomology regions to come close together on each of the 5' side and the 3' side (in the above-described conventional method, it is possible for at least two Cas9s and a pair of microhomology regions to come close together on each of the 5' side and the 3' side). To put it differently, by three Cas9s and a pair of microhomology regions coming close together, it is made easier for the exogenous DNA and the target site to align. Hence, it is surmised that in the gene knock-in method of the present invention in which the number of sequences to be recognized by a guide RNA of the CRISPR-Cas9 is increased on the donor DNA, Cas9s are more likely to interact with each other or are more likely to form a complex although the mechanism is not unclear, and as a result, it is possible for the exogenous DNA and the target site to align at a higher probability, and it is thus possible to insert an exogenous DNA into a target site of a genomic DNA more efficiently than the conventional methods.

### (Cell)

The "cell" in which an exogenous DNA is inserted to edit the DNA, that is, in which the donor DNA and the CRISPR-Cas9 system are introduced in the present invention is not particularly limited, and includes eukaryotic cells and prokaryotic cells (eubacteria cells, archaeal cells). The eukaryotic cells include, for example, animal cells (cells of mammals, fishes, birds, reptiles, amphibians, insects, and the like), plant cells, algal cells, yeast, and the eubacteria cells include Escherichia coli. Salmonella, Bacillus subtilis, lactic acid bacteria, and extreme thermophiles. The archaeal cells include methanogens, Haloarchaea, hyperthermophiles, and thermoacidophiles.

The type of the cell is also not particularly limited, and the animal cells include, for example, reproductive cells such as oocytes and sperm; embryonic cells of the respective stages (for example, one-cell stage embryos, two-cell stage embryos, four-cell stage embryos, eight-cell stage embryos, 16-cell stage embryos, morula stage embryos, and the like); stem cells such as induced pluripotent stem (iPS) cells and embryonic stem (ES) cell; and somatic cells such as fibroblasts, hematopoietic cells, neurons, muscle cells, bone cells, liver cells, and pancreatic cells. These cells may be in the state of being cultured in vitro (for example, cells grown in a medium or on a medium) or may be in the stage of being present in vivo.

### (Genomic DNA)

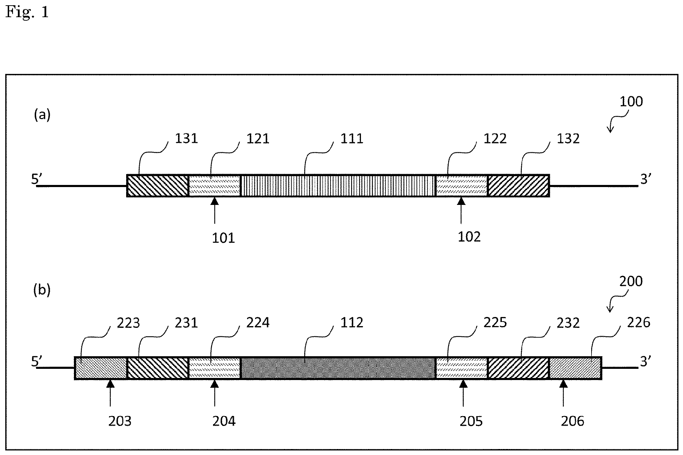

In the present invention, a region containing the target site, which is subjected to the gene knock-in to be achieved on the genomic DNA is referred to as a "target DNA region". In order to indicate the correspondence of the donor DNA and the CRISPR-Cas9 system described below, the target DNA region contains in order from a 5' side: a first nucleotide sequence, a first cleavage target region, the target site, a second cleavage target region, and a second nucleotide sequence for the sake of convenience. In addition, the first cleavage target region includes a first cleavage target portion (indicating a portion between a base, which is to be cleaved by the CRISPR-Cas9 system, and the adjacent base. The same applies below) and a first guide RNA-target sequence, and the second cleavage target region includes a second cleavage target portion and a second guide RNA-target sequence. (a) of Fig. 1 shows a schematic diagram representing an example of a mode of the target DNA region of the genomic DNA according to the present invention.

Although in (a) of Fig. 1, the regions in the target DNA region (100) are illustrated as being directly adjacent to one another sequentially from the 5' side, a pair of the first nucleotide sequence (131) and the first cleavage target region(121), a pair of the first cleavage target region(121) and the target site(111), a pair of the target site(111) and the second cleavage target region(122), and a pair of the second cleavage target region(122) and the second nucleotide sequence (132) each may be independently directly adjacent to each other as in (a) of Fig. 1, or may be partially overlapped in some bases (duplicated bases), or may be adjacent to each other with some bases (intervening bases) in between.

In the case where regions are overlapped in some bases, the number of bases of the duplicated bases is each independently 1 to 5 bases, preferably 1 to 4 bases, and more preferably 1 to 3 bases. In addition, in the case where regions are adjacent to each other with some bases in between, the number of bases of the intervening bases is each independently 1 to 5 bases, preferably 1 to 4 bases, and more preferably 1 to 3 bases.

The first nucleotide sequence and the second nucleotide sequence are each a sequence to which the first' nucleotide sequence and the second' nucleotide sequence of the donor DNA described below can be joined by the microhomology-mediated end joining, respectively. The numbers of bases of the first nucleotide sequence and the second nucleotide sequence are each independently 5 to 40 bases, preferably 5 to 30 bases, and more preferably 5 to 20 bases.

In the first cleavage target region and the second cleavage target region, the first cleavage target portion and the second cleavage target portion each independently may not be on the first guide RNA-target sequence and on the second guide RNA-target sequence, but preferably are on the respective guide RNA-target sequences.

The first guide RNA-target sequence and the second guide RNA-target sequence are sequences that the first guide RNA and second guide RNA of the CRISPR-Cas9 system recognize, respectively (more specifically, complementary sequences for sequences that the first guide RNA and the second guide RNA recognize and bind (complementary strands)). The numbers of bases of the first guide RNA-target sequence and the second guide RNA-target sequence are each independently 23 to 40 bases, preferably 23 to 30 bases, and more preferably 23 to 24 bases. Note that the first guide RNA-target sequence and the second guide RNA-target sequence are preferably selected such that there is no identical sequence except for the target DNA region on the target genomic DNA (there is no sequence having the identity of more than 50%).

The target site is a site in which the insertion of an exogenous DNA is desired, and may be selected from the whole or part of a sequence encoding a gene, the whole or part of a functional region of a promoter or the like, and the like. The target site is not particularly limited, and may be one point between a certain base and the adjacent base, but normally a region with 500 bases or more, preferably 500 to 1000 bases, and more preferably 500 to 700 bases.

Such a target DNA region is not particularly limited, but the CRISPR-Cas9 system is designed to contain a PAM sequence of the Cas9 protein described below that can cleave the first cleavage target region and the second cleavage target region. The first cleavage target region and the second cleavage target region are each preferably selected from regions adjacent to the PAM (proto-spacer adjacent motif)sequence on the 3' side. Note that as described later, it is also possible to modify the recognition specificity of the PAM in accordance with the nucleic acid sequence of the target DNA region by modifying the Cas9 protein (for example, introducing a mutation).

As the method for selecting a target DNA region containing a PAM sequence of a Cas9 protein, various methods have been known, and the method may be determined by utilizing, for example, CRISPR Design Tool (http://crispr.mit.edu/) (Massachusetts Institute of Technology), E-CRISP (http://www.e-crisp.org/E-CRISP/), Zifit Targeter (http://zifit.partners.org/ZiFiT/) (Zing Finger Consortium), Cas9 design (http://cas9.cbi.pku.edu.cn/) (Peking University), CRISPRdirect (http://crispr.dbcls.jp/) (University of Tokyo), CRISPR-P (http://cbi.hzau.edu.cn/crispr/) (Huazhong Agricultural University), Guide RNA Target Design Tool (https://wwws.blueheronbio.com/external/tools/gRNASrc. jsp) (Blue Heron Biotech), and the like.

### (Donor DNA)

In the present invention, the donor DNA is a DNA used for inserting a desired exogenous DNA (knock-in) into the target DNA region cleaved by the Cas9 protein.

The donor DNA is designed in conformity with the target site, and contains in order from the 5' side: a 5'-microhomology region formed of a first' nucleotide sequence that is capable of being joined to a first nucleotide sequence of the genomic DNA by microhomology-mediated end joining; the exogenous DNA; and a 3'-microhomology region formed of a second' nucleotide sequence that is capable of being joined to a second nucleotide sequence of the genomic DNA by microhomology-mediated end joining. More specifically with the correspondence to the CRISPR-Cas9 system described below, the donor DNA contains in order from the 5' side: a third cleavage target region; the 5'-microhomology region; a fourth cleavage target region; the exogenous DNA; a fifth cleavage target region; the 3'-microhomology region; a sixth cleavage target region; and a PAM sequence of a Cas9 protein. In addition, the third cleavage target region is formed of a third cleavage target portion and a third guide RNA-target sequence, the fourth cleavage target region is formed of a fourth cleavage target portion and a fourth guide RNA-target sequence, the fifth cleavage target region is formed of a fifth cleavage target portion and a fifth guide RNA-target sequence, and the sixth cleavage target region is formed of a sixth cleavage target portion and a sixth guide RNA-target sequence. (b) of Fig. 1 shows a schematic diagram showing an example of a mode of the genomic DNA according to the present invention.

Although in (b) of Fig. 1, the regions in the donor DNA (200) are configured as being directly adjacent to one another sequentially from the 5' side, a pair of the third cleavage target region (223) and the 5'-microhomology region (231), a pair of the 5'-microhomology region (231) and the fourth cleavage target region (224), a pair of the fourth cleavage target region (224) and the exogenous DNA (112), a pair of the exogenous DNA (112) and the fifth cleavage target region (225), a pair of the fifth cleavage target region (225) and the 3'-microhomology region (232), and a pair of the 3'-microhomology region (232) and the sixth cleavage target region (226) each may be independently directly adjacent to each other as in (b) of Fig. 1, or may be partially overlapped in some bases (duplicated bases), or may be adjacent to each other with some bases (intervening bases) in between.

In the case where regions are overlapped in some bases, the number of bases of the duplicated bases is each independently preferably 1 to 17 bases. In addition, in the case where regions are adjacent to each other with some bases in between, the number of bases of the intervening bases is each independently preferably 1 to 23 bases.

The 5'-microhomology region is formed of the first' nucleotide sequence that is capable of being joined to the first nucleotide sequence of the genomic DNA by microhomology-mediated end joining, and the 3'-microhomology region is formed of the second' nucleotide sequence that is capable of being joined to the second nucleotide sequence of the genomic DNA by microhomology-mediated end joining. In the present invention , the statement that a specific sequence A and a specific sequence B are "capable of being joined by microhomology-mediated end joining" means that the complementary sequence of the sequence A and the sequence B are capable of forming a complementary strand, that is, the sequence A and the sequence B are identical. For this reason, the first nucleotide sequence and the first' nucleotide sequence are preferably identical, and independently of this, the second nucleotide sequence and the second' nucleotide sequence are preferably identical. In addition, these do not have to have an identity of 100%, and may have an identity of 95% or more, preferably 97% or more, more preferably 99% or more, and further preferably 99.9% or more when calculated using, for example, BLAST (for example, with parameters of the default, that is, the initial setting) or the like. In addition, the first' nucleotide sequence and the second' nucleotide sequence may be identical or different, but are preferably different (for example, the identity is 50% or less) from the viewpoint of efficiency.

The numbers of bases of the first' nucleotide sequence and the second' nucleotide sequence only have to be those that allow microhomology-mediated end joining, and are each independently 5 to 40 bases, preferably 5 to 30 bases, and more preferably 5 to 20 bases.

In the third to sixth cleavage target regions, the third to sixth cleavage target portions may be but do not have to be each independently on the third to sixth guide RNA-target sequences but are preferably on the respective guide RNA-target sequences.

The third to sixth guide RNA-target sequences are sequences which the third to sixth guide RNAs of the CRISPR-Cas9 system described below recognize (or more specifically complementary sequences of sequences which the third to sixth guide RNAs recognize and bind to (complementary strand)), respectively. The numbers of bases of the third to sixth guide RNA-target sequences are each independently 23 to 40 bases, preferably 23 to 30 bases, and more preferably 23 to 24 bases.

In addition, the fourth and fifth guide RNA-target sequences are preferably identical to the first and second guide RNA-target sequences on the target DNA region (sequences having the identity of 100%, or 97%, or 99% or more, or 99.9% or more), but the third and sixth guide RNA-target sequences preferably do not have identical sequences on the target DNA region (there is no sequence having the identity of more than 50%). In the case where there are sequences identical to the third and sixth guide RNA-target sequences on the target DNA region, there is a probability that the mechanism of gene knock-in action by microhomology-mediated end joining does not function. In addition, the third to sixth guide RNA-target sequences are preferably designed to have no identical sequence other than the target DNA region on the target target genomic DNA (have no sequence having the identity of more than 50%).

The exogenous DNA is not particularly limited, and any exogenous DNAs having various sizes can be used. The number of bases of the exogenous DNA (the number of bases of a single chain) is, for example, 100 bases or more, 200 bases or more, 300 bases or more, 400 bases or more, 500 bases or more, 600 bases or more, 700 bases or more, 800 bases or more, 900 bases or more, or 1k bases or more. According to the gene knock-in method of the present invention, it is possible to highly efficiently insert even a long-chain exogenous DNA into the target site of a genomic DNA. The upper limit for the size of the exogenous DNA is not particularly limited, but is, for example, 5k bases or less, 4.5k bases or less, or 4k bases or less. The upper limit may be combined with the lower limit as desired.

The exogenous DNA may be an exogenous DNA that is capable of encoding any desired gene and may be an exogenous DNA that has been codon-optimized as appropriate depending on a cell into which the exogenous DNA is introduced. In addition, the exogenous DNA may have a sequence identical to the sequence of the target site to be replaced (have the identity of 100%, or 97%, or 99% or more, or 99.9% or more), and/or, may have the same number of bases. Moreover, the exogenous DNA may be modified outside the cell (methylation, demethylation, or the like), and for example, an exogenous DNA in which 100%, 99% or more, 98% or more, or 95% or more of the DNA is methylated may be used for the purpose of inserting the exogenous DNA into a promoter of a specific gene. The DNA modification method is not particularly limited, and any publicly-known method can be employed as appropriate. According to the gene knock-in method of the present invention, it is possible to insert even a long-chain exogenous DNA into the target site of a genomic DNA, and it is thus possible to modify a long-chain DNA by modifying the exogenous DNA outside the cell in advance and then introducing the exogenous DNA into the cell.

In addition, in the case where there is a nucleic acid sequence that is desirably removed after the modification in the exogenous DNA, for example, the loxP sequences or the FRT sequences may be added to both ends of the nucleic acid sequence. The nucleic acid sequence flanked by the loxP sequences or the FRT sequences can be removed by reacting the Cre recombinase or the FLP recombinase. In addition, for the purpose of confirming the success of the knock-in, for example, a selectable marker sequence(for example, a fluorescent protein, a drug-resistant gene, or the like) may be added.

In addition, to the exogenous DNA, a promoter and/or another control sequence may be activatably joined. The expression "activatably joined" means that a promoter and a desired DNA (gene) are joined such that the desired DNA joined downstream of the promoter can be expressed. The promoter and/or the other control sequence are not particularly limited, another regulatory element (for example, a terminator sequence) and the like such as a constitutive promoter, a tissue-specific promoter, a time-specific promoter, an inducible promoter, or a CMV promoter can be selected as appropriate.

As the donor DNA used for the gene knock-in method of the present invention, one type may be used alone. In the case of using multiple types of guide RNAs having different target DNA regions, multiple types having exogenous DNAs and microhomology regions corresponding to the respective target DNA regions may be used. In this case, exogenous DNAs contained in the multiple donor DNAs may be DNAs having different nucleic acid sequences.

The donor DNA is not particularly limited, and can be prepared by a conventional publicly-known method or a method according to the publicly-known method. Such a preparation method includes, for example, a method that chemically synthesizes a donor DNA using a commercially-available synthesizer from a first' nucleotide sequence, a second' nucleotide sequence, and sequences of third to sixth cleavage target regions based on the nucleic acid sequence of the target DNA region; and a method that prepares a donor DNA by using a PCR method while adding a first' nucleotide sequence, a second' nucleotide sequence and/or third to sixth cleavage target regions as necessary and using the target DNA region as a template, and further modifies the donor DNA as necessary (for example, by methylating (adding -CH₃ to) cytosine by using CpG methyltransferase.

### (CRISPR-Cas9 System)

The CRISPR-Cas9 system for use in the gene knock-in method of the present invention contains at least a Cas9 protein and a guide RNA for the Cas9 protein as constituent elements.

### [Cas9 protein]

In the CRISPR-Cas9 system according to the present invention, the Cas9 protein is not particularly limited, and includes, for example, a Cas9 protein (SpCas9) derived from Streptococcus pyogenes, a Cpf1 protein (FnCpf1) derived from Francisella novicida, a Cas9 protein (SaCas9) derived from Staphylococcus aureus, and a Cas9 protein (CjCas9) derived from Campylobacter jejuni. Besides, Cas9 proteins derived from various origins are publicly known, and the amino acid sequences and the nucleic acid sequences of Cas9 proteins are registered in public databases, for example, GenBank (http://www.ncbi.nlm.nih.gov) and any of these can be utilized in the present invention as appropriate.

In addition, the Cas9 protein may be a homolog of the above-described publicly-known Cas9 protein, or a mutant having one or more mutations (for example, addition, deletion, replacement, or a combination of these), or a partial peptide, or one containing a modified amino acid sequence, as long as it has the endonuclease activity. Moreover, the Cas9 protein may be a Cas9 protein in which a mutation for modifying the PAM recognition (Benjamin, P. et. al., Nature 523, 481-485 (2015), Hirano, S. et. al., Molecular Cell 61, 886-894 (2016)) or a mutation for the suppression of the off-target activity or functional modification is introduced. In addition, the Cas9 protein may be one encoded in a sequence contained in a commercially-available commercially-available vector.

As the Cas9 protein for use in the gene knock-in method of the present invention, 2 or more Cas9 proteins may be used in combination depending on the guide RNA described below, but one Cas9 protein is preferably used alone from the viewpoint of easiness.

Among these, as the Cas9 protein for use in the gene knock-in method of the present invention, a protein containing point mutations of N692A, M694A, Q695A, and H698A in a Cas9 protein (SpCas9) derived from a wild-type Streptococcus pyogenes (hereinafter sometimes referred to as MSpCas9") is preferable. The nucleic acid sequence of a polynucleotide encoding the MSpCas9 is shown in SEQ ID NO: 1. The PAM (proto-spacer adjacent motif) sequence of the SpCas9 and the MSpCas9 are "5'-NGG (N is any base)-3'". However, it is also possible to modify the recognition specificity of the PAM, and this makes it possible to expand the options for the target DNA region.

### [Guide RNA]

In the CRISPR-Cas9 system according to the present invention, the guide RNA is a combination of a tracrRNA (trans-activating crRNA) and a crRNA (CRISPR RNA) having a nucleic acid sequence complementary to a sequence (a complementary sequence of a guide RNA-target sequence (complementary strand)) on the target DNA.

The guide RNA of the Cas9 protein according to the present invention contains a nucleic acid sequence (hereinafter sometimes referred to as "targeting nucleic acid sequence") complementary to the complementary sequence of each of the guide RNA-target sequences of the target DNA region and the donor DNA in the crRNA.

The crRNA further contains a nucleic acid sequence that is capable of interacting (hybridizing) with the tracrRNA on the 3' side. On the other hand, the tracrRNA contains a nucleic acid sequence that is capable of interacting (hybridizing) with a nucleic acid sequence of part of the crRNA on the 5' side. For this reason, the guide RNA forms a double-stranded RNA that interacts with the Cas9 protein through the interaction with these nucleic acid sequences. Hence, in the gene knock-in method of the present invention, introducing the CRISPR-Cas9 system (the Cas9 protein and the guide RNA for the Cas9 protein) into a cell allows the guide RNA to recognize the respective guide RNA-target sequences of the target DNA region and the donor DNA, that is, bind to the respective complementary sequences (complementary strands) of the guide RNA-target sequences, induce the Cas9 protein, which forms a complex with the guide RNA, into the respective cleavage target regions of the target DNA region and the donor DNA, and then, the Cas9 protein thus induced cleaves the respective cleavage target portions of the target DNA region and the donor DNA through the endonuclease activity.

The guide RNA of the CRISPR-Cas9 system according to the present invention may be a single guide RNA (sgRNA) containing the crRNA and the tracrRNA or a dual guide RNA formed of a crRNA fragment and a tracrRNA fragment.

In each of the target DNA region and the donor DNA, the target DNA cleavage portion (cleavage target portion) does not have to be contained in the guide RNA-target sequence, but is preferably contained in the guide RNA-target sequence. Typically, the cleavage of the target DNA region and donor DNA occurs at positions that are determined by both of the complementarity of base pair formation between the targeting nucleic acid sequence of the guide RNA and the complementary sequence of the guide RNA-target sequence as well as the PAM sequence present on the 3' side. In addition, each entire guide RNA-target sequence is preferably set such that the PAM sequence and a sequence up to the cleavage target portion adjacent to the PAM sequence are lost by the cleavage by the Cas9 protein so as not to undergo another cleavage again by the Cas9 protein present in the cell. In the case of the SpCas9 protein and the MSpCas9 protein, the portions (cleavage target portions) where cleavage occurs in the target DNA region and the donor DNA are generally 3 bases upstream of the PAM sequence (on the 5' terminal side).

In the gene knock-in method of the present invention, the guide RNA contains a first guide RNA, a second guide RNA, a third guide RNA, a fourth guide RNA, a fifth guide RNA, and a sixth guide RNA that respectively recognize a first guide RNA-target sequence and a second guide RNA-target sequence of the genomic DNA as well as a third guide RNA-target sequence, a fourth guide RNA-target sequence, a fifth guide RNA-target sequence, and a sixth guide RNA-target sequence of the donor DNA.

The targeting nucleic acid sequences of the first to sixth guide RNAs may be identical to one another (having the identity of 100%, or 97%, or 99% or more, or 99.9% or more) or may be different from each other. However, it is preferable that at least the targeting nucleic acid sequences of the first to second guide RNAs and the targeting nucleic acid sequences of the third and sixth guide RNA be different from each other (the identity is 50% or less) and the targeting nucleic acid sequence of the first and second guide RNAs be different from each other (the identity is 50% or less) . In addition, it is further preferable that the targeting nucleic acid sequences of the first and fourth guide RNAs be identical to each other (the identity is 100%, or 97%, or 99% or more, or 99.9% or more) and the targeting nucleic acid sequences of the second and fifth guide RNAs be identical to each other (the identity is 100%, or 97%, or 99% or more, or 99.9% or more).

### (Introduction of Donor DNA and CRISPR-Cas9 System Into Cell)

In the gene knock-in method of the present invention, the donor DNA to be introduced into a cell may be in the form of the donor DNA itself (DNA fragment) or may be in the form of a vector containing the DNA fragment.

In addition, in the gene knock-in method of the present invention, for the CRISPR-Cas9 system to be introduced into a cell, the Cas9 protein may be in the form of a protein, or may be in the form of an RNA or DNA (polynucleotide) encoding the protein, or may be in the form of a vector (expression vector) expressing the protein. In addition, independently from this, the guide RNA may be in the form of an RNA, or may be in the form of a DNA (polynucleotide) encoding the RNA, or may be in the form of a vector (expression vector) expressing the RNA.

In the case of employing the form of an expression vector in the introduction of the CRISPR-Cas9 system, for example, a vector expressing the Cas9 protein and a vector expressing the guide RNA may be introduced into the cell, or a vector expressing both of the Cas9 protein and the guide RNA may be introduced into the cell. In the case of using multiple Cas9 proteins, polynucleotides encoding the respective Cas9 protein may be inserted into the same expression vector or may be inserted into separate expression vectors. In addition, polynucleotides encoding the respective multiple guide RNAs may be inserted into the same expression vector or may be inserted separate expression vectors.

In addition, in the case of employing the form of an expression vector in the introduction of the CRISPR-Cas9 system, the polynucleotide encoding the Cas9 protein may be a polynucleotide that is codon-optimized as appropriate depending on the cell into which the CRISPR-Cas9 system is introduced. In addition, it is preferable that the expression vector contain a promoter and/or another control sequence activatably joined to the polynucleotide to be expressed. Moreover, it is preferable that the expression vector be capable of stably expressing the protein to be encoded without being incorporated into a host genome. Such an expression vector can be fabricated in accordance with the conventional publicly-known method as appropriate.

As the method for introducing the donor DNA and the CRISPR-Cas9 system into the cell, a publicly-known method for introducing a protein, a DNA, an RNA fragment, and the like into a cell can be employed as appropriate depending on the type of the cell. Such method includes, for example, an electroporation method, a microinjection method, a particle gun method, a calcium phosphate method, a polyethyleneimine (PEI) method, liposome method (lipofection method), a DEAE-dextran method, a cationic lipid-mediated transfection, viruses (adenoviruses, lentiviruses, adeno-associated viruses, Baculoviruses, and the like), an agrobacterium method, a lithium acetate method, a spheroplast method, a heat shock method (calcium chloride method, rubidium chloride method), and the like. Such methods are described in many standard laboratory manuals such as "Leonard G.Daviset al., Basic methods in molecular biology, New York: Elsevier, 1986".

Once the donor DNA and the CRISPR-Cas9 system are introduced into the cell, the first to second guide RNAs of the CRISPR-Cas9 system bind respectively to the complementary sequences (complementary strands) of the first to second guide RNA-target sequences of the target DNA region, and the third to sixth guide RNAs bind respectively to the complementary sequences (complementary strands) of the third to sixth guide RNA-target sequences of the donor DNA. Moreover, the first to sixth guide RNAs form complexes with the Cas9 proteins to induce the Cas9 proteins into the first to sixth guide RNA-target sequences (first to sixth cleavage target regions). In addition, the Cas9 proteins thus induced cleave the first to sixth cleavage target portions in the first to sixth cleavage target regions by their endonuclease activity, respectively. At this time, the first nucleotide sequence and the second nucleotide sequence, which are the cleaved ends of the target DNA region, are aligned with the first' nucleotide sequence and the second' nucleotide sequence of the donor DNA by microhomology-mediated end joining. Moreover, since around these microhomologies, three Cas9 proteins have been induced on each of the 5' side (first nucleotide sequence and first' nucleotide sequence) and the 3' side (second nucleotide sequence and second' nucleotide sequence) by the first, third, and fourth guide RNAs and by the second, fifth, and sixth guide RNAs, the exogenous DNA is inserted between the first nucleotide sequence and the second nucleotide sequence of the target DNA region.

In the gene knock-in method of the present invention, it has been confirmed that although the mechanism is unclear, the exogenous DNA is inserted between the first nucleotide sequence and the second nucleotide sequence of the target DNA region more efficiently than the conventional methods (for example, while NPL 3 states that the knock-in efficiency is 20% approximately, the knock-in efficiency was 31.6% in Examples described below), it is considered that one of the reasons is that increasing the sequences (guide RNA-target sequences) to be recognized by the guide RNA of the CRISPR-Cas9 makes it easier for the Cas9 proteins to interact with each other or form complexes.

### <Gene Knock-in Cell Fabrication Method, Non-human Individual Fabrication Method, Gene Knock-in Cell>

The present invention provides a method for fabricating a cell (gene knock-in cell) in which the exogenous DNA is inserted into the target site. This method includes a step of obtaining the gene knock-in cell by the above-described gene knock-in method.

The present invention also provides a method for fabricating a non-human individual containing a cell in which the exogenous DNA is inserted into the target site. This method includes a step of fabricating a non-human individual from a cell obtained by the above-described gene knock-in method or the above-described gene knock-in cell fabrication method. The non-human individual includes, for example, a non-human animal and a plant. The non-human animal includes mammals (mouse, rat, guinea pig, hamster, rabbit, human, monkey, pig, bovine, goat, sheep, and the like), fishes, birds, reptiles, amphibians, and insects, but is preferably a rodent such as mouse, rat, guinea pig, and hamster, and is particularly preferably a mouse. The plant includes, for example, grains, oil crops, forage crops, fruits, and vegetables. Specific crops include, for example, rice, corn, banana, peanut, sunflower, tomato, turnip rape, tobacco, wheat, barley, potato, soybean, cotton, and carnation.

As the method for fabricating a non-human individual from the gene knock-in cell, a publicly-known method can be used. In the case where a non-human individual is fabricated from a cell in an animal, a reproductive cell or a pluripotent stem cell is generally used. for example, the donor DNA and the CRISPR-Cas9 system are microscopically injected into oocytes and the oocytes thus obtained are transplanted into a uterus of a pseudopregnant female non-human mammal, and thereafter an offspring can be obtained. On the other hand, in the case of plants, it has been known from a long time ago that somatic cells of plants have totipotency. For example, the donor DNA and the CRISPR-Cas9 system are microscopically injected into plant cells and a plant is regenerated from the plant cells thus obtained, so that a plant in which a desired DNA has been subjected to knock-in can be obtained. In addition, a progeny or clone in which a desired DNA has been subjected to knock-in can be obtained from the non-human individual obtained.

Confirmation of the presence or absence of gene modification and determination of the gene type may be made based on the conventional publicly-known approaches, and for example, the PCR method, the sequence determination methods, and the Southern blotting method and the like can be used.

According to the gene knock-in method of the present invention, it is possible to insert a long-chain (for example, 700 bp or more) exogenous DNA and/or an exogenous DNA modified outside the cell into a target site of a genomic DNA highly efficiently. Hence, for example, by using and inserting an exogenous DNA containing 700 bp or more 100% methylated base pairs into a promoter of a specific gene, it is possible to obtain a gene knock-in cell in which the expression of the gene has been suppressed with high accuracy.

### <Malignant Transformation Risk Evaluation Method>

The present invention provides a malignant transformation risk evaluation method (hereinafter sometimes referred to simply as "evaluation method") for evaluating malignant transformation risk of a target cell, the method comprising:
a methylation step of methylating a target promoter site contained in a promoter of a genomic DNA in the target cell;
a detection step of detecting malignant transformation of the target cell; and
a cell evaluation step of evaluating malignant transformation risk of the cell by using the malignant transformation of the target cell as an indicator.

### (Target Cell)

The "target cell" according to the evaluation method of the present invention indicates a cell to be subjected to the evaluation method of the present invention, and includes cells given as the "cell" in which DNA is to be edited by inserting the above-described exogenous DNA. Among these, for example, cells of humans and non-human animals (for example, mammals (mouse, rat, guinea pig, hamster, rabbit, monkey, pig, bovine, goat, sheep, and the like), fishes, birds, reptiles, amphibians, and insects) are preferable, and cells of humans and non-human mammals are more preferable. In addition, although the type of the target cell according to the evaluation method of the present invention also includes the above-described types, among these, a cell sampled from a living organism and/or a cell cultured in vitro (for example, a cell grown in a medium or on a medium) is preferable. The evaluation method of the present invention can be conducted on these cells in vitro.

### (Methylation Step)

In the evaluation method of the present invention, first, a target promoter site contained in a promoter of a genomic DNA is methylated in the target cell (methylation step). Note that in the evaluation method of the present invention, the "methylation of DNA" means methylation of cytosine.

In the evaluation method of the present invention, the "target promoter site" indicates a site to be methylated that is contained in any desired promoter on a genomic DNA in the target cell, and may be the whole or part of the entire sequence of the promoter. However, in the promoter, the length of the sequence to be methylated (that is, the length of the target promoter site) is preferably 500 bp or more, and more preferably 700 bp or more.

In addition, it is preferable that the target promoter site to be methylated in the evaluation method of the present invention contain 100% of up to 500 bp upstream of a transcription start site of a gene controlled by a promoter containing the target promoter site. That is, in the methylation according to the evaluation method of the present invention, it is preferable that 100% of a promoter up to 500 bp upstream of the transcription start site be methylated. If the range of the target promoter site to be methylated is less than the lower limit, the expression of the gene controlled by the promoter containing the target promoter site cannot be completely controlled, so that the evaluation accuracy tends to decrease. The upper limit value for the length of the target promoter site according to the evaluation method of the present invention is not particularly limited, but is for example, 5 kbp or less, 4.5 kbp or less, 4 kbp or less, or 1 kbp or less. The upper limits can each be combined with the lower limit as desired.

The method for methylation (methylation method) according to the evaluation method of the present invention is not particularly limited as long as the method is capable of methylating a long-chain target promoter site that satisfies the above-described conditions in the target cell. Such a method includes, for example, a method for knocking in an exogenous methylated promoter site to the target promoter site using the methods described in Sakuma et al., Nat protocol, Vol. 11 No. 1, 2016, p. 118-133 (NPL 1), Katayama et al., Angew Chem Int Ed, 55(22), 2016, p. 6452-6456 (NPL2), Katayama et al., Life Sci Alliance, Vol. 3 No. 1 ,2019, e201900528 (NPL3), and the like or a method in accordance with the methods; a method for knocking in an exogenous methylated promoter site to the target promoter site by using the above-described gene knock-in method of the present invention; and a method using microhomology-mediated end joining (MMEJ) .

Among these, as the methylation method according to the evaluation method of the present invention, the use of the above-described gene knock-in method of the present invention is preferable from the viewpoint that it is possible to insert the whole or part (in the Specification, sometimes referred to as a "methylated promoter site") of a longer-chain, methylated promoter into a target promoter site of a genomic DNA as an exogenous DNA more efficiently than the conventional methods. That is, the evaluation method of the present invention is preferably a method in which the methylation step is a step of knocking in an exogenous methylated promoter site into the target promoter site of the genomic DNA in the target cell by using the gene knock-in method of the present invention, the target site is the target promoter site, and the exogenous DNA is the methylated promoter site.

In the case where the methylation step according to the evaluation method of the present invention is a step using the gene knock-in method of the present invention, in the methylation step, the "cell" in the gene knock-in method is the target cell; the "genomic DNA" is a genomic DNA of the target cell; the "target site" is the target promoter site; the "exogenous DNA" is the "methylated promoter site"; and the "donor DNA" is a DNA containing the methylated promoter site.

In the evaluation method of the present invention, in the case where the methylation step is a step using the gene knock-in method of the present invention, the target promoter site is a site that is replaced with the methylated promoter site. In addition, the methylated promoter site indicates a sequence formed by methylating a sequence identical to that of the target promoter site or a sequence formed by methylating part of the sequence of the target promoter site, and is a DNA to be inserted into the genomic DNA in place of the target promoter site by the gene knock-in method.

According to the gene knock-in method of the present invention, it is possible to insert a long-chain (for example, 700 bp or more) methylated promoter site (exogenous DNA) into a target promoter site (target site) of a genomic DNA highly efficiently, making it possible to insert a methylated promoter site formed of 700 bp or more 100%-methylated base pairs into the target promoter site, for example.

The methylated promoter site may be codon-optimized as appropriate depending on a cell into which the methylated promoter site is introduced. In addition, the sequence may be different from the sequence of the target promoter site to be replaced, but is preferably identical (the identity is 100%, or 97%, or 99% or more, or 99.9% or more) . In addition, the length of the methylated promoter site is preferably equal to the length of the target promoter site to be replaced, more preferably 500 bp or more, and further preferably 700 bp or more. In addition, it is preferable that 98% or more and more preferably 100% of the methylated promoter site be methylated. The upper limit value for the length of the methylated promoter site is not particularly limited, but is for example, 5 kbp or less, 4.5 kbp or less, 4 kbp or less, or 1 kbp or less. The upper limits can each be combined with the lower limit as desired.

The methylated promoter site can be obtained, for example, by preparing a DNA by a method that chemically synthesizes the DNA using a commercially-available synthesizer from the target promoter site as well as a first' nucleotide sequence, a second' nucleotide sequence, and sequences of third to sixth cleavage target regions based on nucleic acid sequences of a first nucleotide sequence on the 5' side and a second nucleotide sequence on the 3' side of the target promoter site; a method that prepares the DNA by using the PCR method while adding a first' nucleotide sequence, a second' nucleotide sequence and/or third to sixth cleavage target regions as necessary and using the target promoter site as a template; or the like, and then methylating the prepared DNA (for example, methylating (adding -CH₃ to) cytosine by using CpG methyltransferase, or the like).

### (Detection Step)

In the evaluation method of the present invention, subsequently, malignant transformation of the target cell is detected (detection step). In the present invention, the "malignant transformation of a cell" means the state where cells abnormally proliferate (cancer), and particularly in the present invention, means that cells fall into this state due to accumulation of DNA methylation to a promoter or suppression of the expression of the gene controlled by the promoter. As a detection method for detecting such malignant transformation of cells includes, for example, a method for detecting cancer cells after the methylation step. Note that the method for detecting a cancer cell includes not only a method for detecting the presence or absence of cancer cells but also a method for quantifying the cancer cells.

The method for detecting a cancer cell is not particularly limited, and, a method in accordance with conventional publicly-known methods can be employed as appropriated depending on the type of the target cell and the like. For example, in the case where the target cell is a B cell, there is a method that cultures the target cell after the methylation step in an agar gel medium and detects viable cells. When the target cell after the methylation step is cultured in an agar gel medium, if the B cells have not been turned into cancer through malignant transformation, the B cells cannot survive in the gel but are killed. If the B cells are turned into cancer cells through malignant transformation, the B cells form spheroids and can survive. Hence, it is possible to detect the malignant transformation of the target cell by detecting the viable cells. The method for detecting viable cells is also not particularly limited, and for example, viable cells can be detected through staining with Crystal Violet or the like, and may be quantified as necessary.

Besides, the detection method includes, for example, immunostaining methods using the anti-CD20 antibody, the anti-CD10 antibody, the anti-BCL-6 antibody, the anti-MUM-1 antibody, and combinations of these antibodies.

### (Evaluation Step)

In the evaluation method of the present invention, subsequently, the malignant transformation risk of the cell is evaluated by using the malignant transformation of the target cell as an indicator (cell evaluation step) .

In the present invention, the "malignant transformation risk of a cell" indicates a probability that the target cell turns into cancer (preferably, turns into cancer due to accumulation of DNA methylation to a promoter or suppression of the expression of the gene controlled by the promoter). In the present invention, the evaluation of the malignant transformation risk is conducted such that if the malignant transformation of the target cell is detected, it is determined that there is probability or high probability that the cell turns into cancer through malignant transformation, and the cell is screened from the group having no or low probability. On the other hand, if the malignant transformation of the target cell is not detected, it is determined that there is no or low probability that the cell turns into cancer through malignant transformation, and the cell is screened from the group having probability or high probability. In the present invention, the determination of the probability includes not only determination on if there is a probability but also evaluation of the degree of the probability (evaluation such as high/medium/low, or the like). In addition, the evaluation method of the present invention also includes screening in which a subject having a malignant transformation risk or high malignant transformation risk is screened from a group having no or low malignant transformation risk.

### (Gene Evaluation Method)

The evaluation method of the present invention may be a method (hereinafter sometimes referred to as "gene evaluation method") further comprising: a gene evaluation step of evaluating malignant transformation capability of a gene controlled by a promoter containing the target promoter site by using the malignant transformation of the target cell as an indicator.

In the present invention, the "malignant transformation capability of a gene" indicates that the gene may be involved in malignant transformation, and includes the fact that the cell may turn into cancer due to accumulation of DNA methylation to a promoter of the gene and the fact that the cell may turn into cancer due to suppression of the expression of the gene. In the present invention, the evaluation of the malignant transformation capability of a gene is conducted such that if the malignant transformation of the target cell is detected, it is determined that there is probability or high probability that the gene controlled by the promoter containing the target promoter site in the target cell is involved in malignant transformation, and the gene is screened from the group having no or low probability. On the other hand, if the malignant transformation of the target cell is not detected, it is determined that there is no or low probability that the gene is involved in malignant transformation, and the gene is screened from the group having probability or high probability.

For example, after methylation is introduced into a promoter of any desired gene A in any desired target cell, if the target cell turns into cancer (if cancer cells are detected), it can be determined that there is probability or high probability that the target cell turns into cancer through malignant transformation, and it can also be determined that there is probability or high probability the gene A is involved in malignant transformation. In addition, the method for detecting malignant transformation is a method capable of quantification, or the like, it may be determined that there is high or low probability that the target cell turns into cancer and that there is high or low probability that the gene A is involved in malignant transformation, depending on the degree of the quantification. The criteria for the determination can be set as appropriate depending on the purpose of the evaluation, the types of a cell and a promoter, and the like.

In the case where the evaluation method of the present invention is the gene evaluation method, the target cell and the promoter may be each independently any desired one depending on the purpose of the evaluation. In this way, for example, by selecting multiple promoters for any desired gene as promoters containing a target promoter site to be methylated, it becomes possible to search for a gene involved in malignant transformation due to accumulation of DNA methylation to a promoter or malignant transformation due to suppression of the expression by using the malignant transformation of a target cell as an indicator.

### (Malignant Transformation Risk Evaluation Method for Test Subject)

Moreover, the evaluation method of the present invention may be made into a method (hereinafter sometimes referred to as "test subject evaluation method") further comprising: a test subject evaluation step of evaluating malignant transformation risk of a test subject by using a cell derived from the test subject as the target cell and the malignant transformation of the target cell as an indicator.

In the present invention, the "malignant transformation risk of a test subject" indicates a probability that the test subject develops cancer (preferably, develops cancer due to accumulation of DNA methylation to a promoter containing the target promoter site or develops cancer due to suppression of the expression of a gene controlled by the promoter containing the target promoter site). Alternatively, in the case where the test subject has already developed cancer, the "malignant transformation risk of a test subject" may indicate a probability that the cancer is a cancer due to accumulation of DNA methylation to a promoter containing the target promoter site or a probability that the cancer is a cancer due to suppression of the expression of a gene controlled by the promoter containing the target promoter site. The cancer is not particularly limited, but includes, for example, blood cancer, liver cancer, stomach cancer, and pancreatic cancer. In addition, in the present invention, the "test subject" is not particularly limited as long as the "test subject" is a human to be subjected to the evaluation method of the present invention. The "test subject" may be, for example, a healthy subject or a asymptomatic subject for the purpose of predicting, screening, or the like of malignant transformation risk, or may be a patient known to have already developed cancer or the like.

In the present invention, the evaluation of the malignant transformation risk of a test subject is conducted such that if the malignant transformation of the target cell is detected, it is determined that there is probability or high probability that the test subject from which the target cell is derived develops the cancer (or has developed the cancer), and the test subject is screened from the group having no or low probability. On the other hand, if the malignant transformation of a target cell is not detected, it is determined that there is no or low probability that the test subject develops the cancer (or has developed the cancer), and the test subject is screened from the group having probability or high probability.

In the case where the evaluation method of the present invention is the test subject evaluation method, the promoter containing the target promoter site needs to be a promoter of a gene with which a cell is known to turn into cancer due to accumulation of methylation to the promoter or suppression of the expression. Such promoter includes, for example, a promoter of at least one gene selected from the group consisting of SP3, CDKN2A, p53, p21, and TERT1, and among these a promoter of at least one gene selected from the group consisting of SP3 and CDKN2A is preferable.

In addition, the target cell in this case includes, for example, at least one selected from the group consisting of a B cell, a liver cell, a gastric epithelial cell, and a mucosal cell of pancreatic duct, and among these, at least one selected from the group consisting of a B cell and a liver cell is preferable. In the test subject evaluation method, the target cell can be obtained from a sample (the blood, a piece of tissue, or the like) collected from the test subject by a publicly-known method as appropriate depending on the purpose of the evaluation method.

Moreover, the combination of the target cell and the gene controlled by the promoter includes, for example, at least one selected from the group consisting of the combination of a B cell and SP3, the combination of a liver cell and CDKN2A, the combination of a gastric epithelial cell and p53, p21, or the like, and the combination of a mucosal cell of pancreatic duct and TERT1, and among these, at least one selected from the group consisting of the combination of a B cell and SP3 and the combination of a liver cell and CDKN2A is preferable.

For example, in the case where after methylation is introduced into a target promoter site contained in a promoter of a SP3 gene in a B cell derived from a test subject, the B cell turns into cancer (a cancer cell is detected), it can be determined that there is probability or high probability that the B cell turns into cancer through malignant transformation, and it can also be determined that there is probability or high probability that the test subject develops cancer related to the B cell and/or SP3 gene (for example, B-cell lymphoma). In addition, in the case where the method for detecting malignant transformation is a method capable of quantification, and the like, it may be determined that there is high or low probability that the test subject develops cancer depending on the degree of the quantification. The criteria for the determination can be set as appropriate depending on the purpose of the evaluation, the types of a cell and a promoter, and the like.

According to the test subject evaluation method, it is possible to directly evaluate the risk of development of cancer due to accumulation of DNA methylation by using a cell of the test subject his/her own, and it becomes possible to achieve early detection of the cancer and prevention of the development by identifying a test subject having a high risk of development of the cancer and conducting periodic medical examination and management. Note that the test subject evaluation method of the present invention may be expressed as a method for assisting diagnosis by a doctor or the like, or a method for providing information for diagnosis by a doctor or the like.

### <Cancer Cell Production Method>

The present invention also provides a cancer cell production method comprising: a step of obtaining a cancer cell by methylating a target promoter site contained in a promoter of a genomic DNA in a cell. This method comprises a methylation step of methylating the target promoter site by means of the above-described methylation step, and the methylation step is as described in the evaluation method of the present invention, including its preferable mode.

In the case where any desired target cell and any desired target promoter site are used as the target cell and the target promoter site, the cancer cell production method of the present invention preferably further comprises the above-described detection step, making it possible to obtain a cell in which malignant transformation has been detected in the detection step as the cancer cell. In addition, in the cancer cell production method of the present invention, it is possible to obtain a cancer cell in which a promoter of a target gene is methylated to cause malignant transformation, by using a promoter containing the target cell and/or the target promoter site as a promoter of a cell and/or a gene known to turn into cancer due to accumulation of DNA methylation. In this case as well, the cancer cell production method may further comprise the detection step.

The present invention also provides a method for fabricating a non-human individual containing a cancer cell in which the target promoter site is methylated. This method comprises a step of fabricating a non-human individual containing a cell obtained in the cancer cell production method. The non-human individual fabrication method is as described above. The non-human individual includes, for example, the non-human animal, and is preferably a rodent such as a mouse, a rat, a guinea pig, and a hamster, and particularly preferably a mouse.

### <Kit>

In addition, the present invention provides a kit for use in the gene knock-in method of the present invention, the gene knock-in cell fabrication method of the present invention, the malignant transformation risk evaluation method of the present invention, the cancer cell production method of the present invention, or the non-human individual fabrication method of the present invention, that is, a kit for use in the gene knock-in method of the present invention.

The kit of the present invention comprises:
at least one selected from the group consisting of
(a) a Cas9 protein cleaving a first cleavage target region and a second cleavage target region of the genomic DNA as well as a third cleavage target region, a fourth cleavage target region, a fifth cleavage target region, and a sixth cleavage target region of the donor DNA, a polynucleotide encoding the Cas9 protein, or a vector expressing the Cas9 protein, and
(b) a guide RNA of the Cas9 protein, a polynucleotide encoding the guide RNA, or a vector expressing the guide RNA. The kit of the present invention may comprise (c) the donor DNA. In addition, (b) a guide RNA of the Cas9 protein, a polynucleotide encoding the guide RNA, or a vector expressing the guide RNA may be (b') a vector containing an insertion site for inserting a guide RNA designed and prepared as appropriate in conformity with a sequence of the target DNA region. These proteins, polynucleotide, and vectors are each independently as described in the above-described gene knock-in method of the present invention.

The kit of the present invention may further comprise one or a plurality of additional reagents. Such additional reagents include, for example, a diluted buffer solution, a reconstitution solution, a wash buffer solution, a nucleic acid transfection reagent, a protein transfection reagent, and a control reagent (for example, a control guide RNA), but are not limited to these. The kit may further comprise an instruction manual for conducting the methods of the present invention.

The elements contained in the kit may be stored in individual containers or may be stored in a single container. Each element may be stored in the container in an amount for a single use, or may be stored in one container an amount for multiple uses. Each element may be stored in a container in dried form or may be stored in a container in a form dissolved in a suitable solvent.

### [Examples]

Hereinafter, the present invention will be described in further detail based on Examples; however the present invention is not limited to Examples described below. In addition, the operations of PCR and the like described below were conducted by conventional methods as appropriate.

### (Example 1)

### (1) CRISPR-Cas9 System

### [MSpCas9]

PCR was conducted using the following primers that were designed to introduce point mutations of N692A, M694A, Q695A, and H698A into a wild-type SpCas9 protein with a PX459 plasmid (GenScript Biotech) expressing the wild-type SpCas9 protein as a template:
a MSpCas9 forward primer (SEQ ID NO: 2), and
a MSpCas9 reverse primer (SEQ ID NO: 3), and KOD ONE PCR Master Mix (Toyobo Co., Ltd.). The PCR product thus obtained was phosphorylated by using a T4 polynucleotide kinase (Toyobo Co., Ltd.), and was ligated by using a Mightly Ligation mix (Takara) to obtain a plasmid (PX459-MSpCas9) expressing a modified Cas9 protein (MSpCas9) containing the point mutations. When the introduction of the mutations was confirmed by the Sanger Sequencing, it was surely confirmed that a nucleotide sequence encoding a protein containing the point mutations in the wild-type SpCas9 protein was obtained (a nucleotide sequence encoding the MSpCas9 protein: SEQ ID NO: 1).

### [Guide RNA]

With 700 bp on the 3' side in the promoter of the human SP3 gene (hSP3) as a target site, polynucleotides respectively encoding a guide RNA 1 (sgRNA1) formed of a nucleic acid sequence (targeting nucleic acid sequence, SEQ ID NO: 4) complementary to a complementary sequence of a guide RNA-target sequence 1 upstream of the PAM sequence (5'-NGG) of the MSpCas9 protein in the region on the 5' side of the leading strand of the target site; a guide RNA 2 (sgRNA2) formed of a nucleic acid sequence (targeting nucleic acid sequence, SEQ ID NO: 5) complementary to a complementary sequence of a guide RNA-target sequence 2 upstream of the PAM sequence of the MSpCas9 protein in the region on the 3' side of the leading strand of the target site; a guide RNA 3 (sgRNA3) formed of a nucleic acid sequence (targeting nucleic acid sequence, SEQ ID NO: 6) complementary to a complementary sequence of a guide RNA-target sequence 3 of a donor DNA described below; and a guide RNA 6 (sgRNA6) formed of a nucleic acid sequence (targeting nucleic acid sequence, SEQ ID NO: 7) complementary to a complementary sequence of a guide RNA-target sequence 6 of the donor DNA described below are synthesized.

The polynucleotide (DNA) encoding the guide RNA 1 and the polynucleotide encoding the guide RNA 3 were inserted into a guide RNA insertion site of plasmid PX459-MSpCas9 to construct a plasmid CRISPR1 (PX459-MSpCas9-sgRNA1-sgRNA3) expressing the MSpCas9 protein, the guide RNA 1, and the guide RNA 3.

In addition, the polynucleotide encoding the guide RNA 2 and the polynucleotide encoding the guide RNA 6 were inserted into a guide RNA insertion site of other plasmid PX459-MSpCas9 to construct a plasmid CRISPR2 (PX459-MSpCas9-sgRNA2-sgRNA6) expressing the MSpCas9 protein, the guide RNA 2, and the guide RNA 6.

### (2) Donor DNA

The 700 bp on the 3' side in the promoter of the hSP3 was amplified by using the following primers:
a donor hSP3pro forward primer (SEQ ID NO: 8), and
a donor hSP3pro reverse primer (SEQ ID NO: 9). A PCR product obtained by using the primers contains, from the 5' side of the leading strand,
the guide RNA-target sequence 3 recognized by the guide RNA 3;
a first' nucleotide sequence (5'-microhomology region, 20 bp, containing the PAM sequence of the MSpCas9 protein) identical (capable of being joined by microhomology-mediated end joining) to a first nucleotide sequence on the 5' side of the target site;
the guide RNA-target sequence 1;
the 700 base (exogenous DNA, containing the PAM sequence of the MSpCas9 protein) on the 3' side of the promoter of the hSP3;
the guide RNA-target sequence 2;
a second' nucleotide sequence (3'-microhomology region, 20 bp, containing the PAM sequence of the MSpCas9 protein) identical (capable of being joined by microhomology-mediated end joining) to the second nucleotide sequence on the 3' side of the target site;
the guide RNA-target sequence 6 recognized by the guide RNA 6; and
the PAM sequence of the MSpCas9 protein. Note that the 5'-microhomology region and the guide RNA-target sequence 1; the 3'-microhomology region and the guide RNA-target sequence 6 are each duplicates of each other. The PCR product was methylated by using CpG methyltransferase (New England Biolabs) to be a donor DNA.

### (3) Cell Culture

First, HEK 293 cells (JCRB cell bank) were maintained and cultured in a medium (10% medium) obtained by adding 10% FBS (Hyclone blood serum, GE Healthcare) and 0.5% Penicillin-Streptomycin (Nacalai tesque, Inc.) to DMEM (Nacalai tesque, Inc.). Subsequently, the maintained and cultured cells were dissociated by 0.25% Trypsin-EDTA (Nacalai tesque, Inc.) and was inoculated into a 24-well plate in 1×10⁵ cells/well.

### (4) Puromycin Selection

From after 24 hours from the inoculation into the 24-well plate, the cells were cultured in a medium to which 0 to 5 µg/mL of puromycin was added. The cells were collected on each of day 2 and day 3 from the start of the addition. Then, Trypan Blue (Wako) was added and the number of viable cells (unstained cells) was counted.

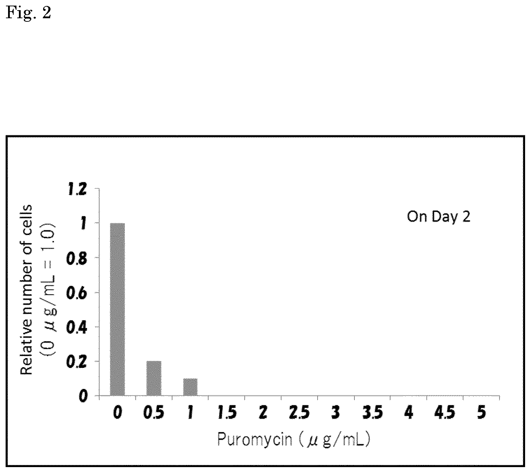

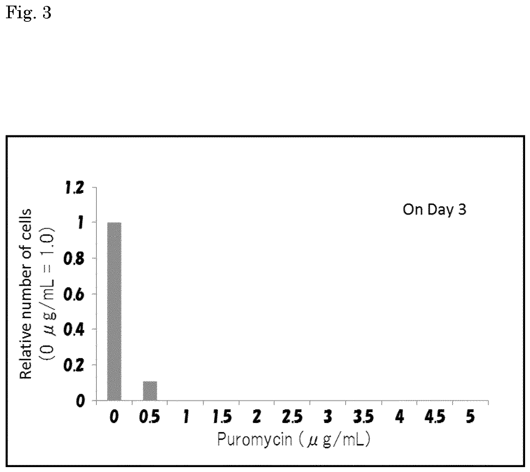

Fig. 2 shows a relation between the concentration of puromycin and the number of viable cells on day 2 from the start of the addition, and Fig. 3 shows a relation between the concentration of puromycin and the number of viable cells on day 3 from the start of the addition. Note that in Figs. 2 to 3, each number of viable cells (vertical axis) was the relative number of cells to the number of viable cells with the concentration of puromycin being 0 µg/mL. As shown in Figs. 2 to 3, the concentration (optimum concentration) of puromycin that was able to kill 90% or more of the HEK 293 cells on day 2 after the addition, and that was able to kill all the HEK 293 cells on day 3 was 1 µg/mL.

### (5) T7EI Assay

HEK 293 cells were inoculated into a 24-well plate as in the above (3), and the plasmid CRISPR1 or CRISPR2 constructed as described above was transfected to be 400 ng/well by using polyethyleneimine (PEI, Polyplus-transfection SA) on the next day following the inoculation. The medium was replaced with the above 10% medium on the next day following the transfection, and culturing was started in a medium to which 1 µg/mL of puromycin was added after 48 hours from the transfection.

From the cells (adherent cells) survived after 48 hours from the start of addition of puromycin, the genomic DNA was extracted by using Qiaamp DNA mini kit (Qiagen). With the genomic DNA as a template, a region containing 700 bp (target site) on the 3' side in the promoter of hSP3 was amplified by PCR using the following primers:
a hSP3pro forward primer (SEQ ID NO: 10), and
a hSP3pro reverse primer (SEQ ID NO: 11).

Then, 200 ng of the PCR product thus obtained, a 10×M buffer (Takara), and DDW were mixed to obtain 10 µL of a reaction mixture in total, which was then reacted under the following conditions of: 95°C and 10 min, 85°C and 1 min, 75°C and 1 min, 65°C and 1 min, 55°C and 1 min, 45°C and 1 min, 35°C and 1 min, and 25°C and 1 min. To 10 µL of the reaction mixture after the reaction, 1.5 µL of 10×NEB2 buffer (New England Biolabs), 0.5 µL of T7 Endonuclease I (T7EI, New England Biolabs), and 3 µL of DDW were added, and T7EI treatment was conducted under the conditions of 37°C and 30 min. After the T7EI treatment, electrophoresis was conducted with 3% agarose gel (Nippon Gene Co., Ltd.).

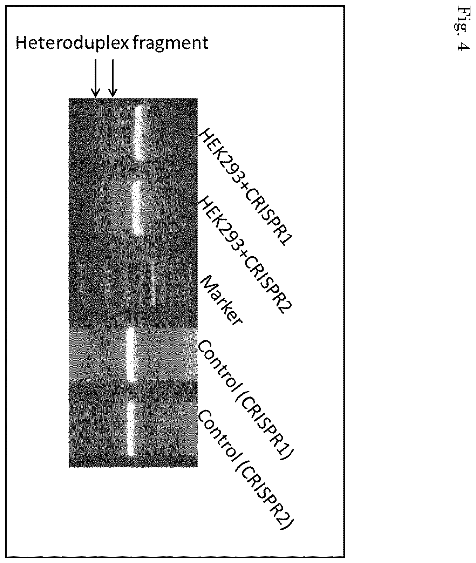

Fig. 4 shows a picture after the electrophoresis (electrophoresis picture). Fig. 4 also shows results (Controls) of conducting electrophoresis on only the plasmid CRISPR1 and the CRISPR2 fragments. As shown in Fig. 4, in the HEK 293 cell in which the plasmid CRISPR1 or CRISPR2 was introduced, a heteroduplex fragment formed between the wild-type DNA and the mutation DNA was confirmed, and it was confirmed that the CRISPR-Cas9 system introduced by each of the plasmid CRISPR1 and CRISPR2 was able to correctly cleave the guide RNA-target sequence 1 on the 5' side and the guide RNA-target sequence 2 on the 3' side of the target site.

### (6) Gene Knock-in

HEK 293 cells were inoculated into a 24-well plate as in the above (3), and 150 ng/well of the plasmid CRISPR1 and 150 ng/well of the plasmid CRISPR2 and 150 ng/well of the donor DNA constructed as described above were combined and transfected by using polyethyleneimine (PEI, Polyplus-transfection SA) on the next day following the inoculation. The medium was replaced with the above 10% medium on the next day following the transfection, and culturing was started in a medium to which 1 µg/mL of puromycin was added after 48 hours from the transfection.

The cell survived after 48 hours from the start of addition of puromycin were dissociated by 0.25% Trypsin-EDTA (Nacalai tesque, Inc.) and the cells were diluted to be 25 cells/10 mL (limiting dilution). The cells thus diluted were inoculated in the wells of a 96 well plate in an amount of 200 µL each, followed by culturing for 10 to 12 days to establish single-cell clones.

### (7) RT-PCR

First, RNAs were extracted from the single-cell clones established in the above (6) by using QIAZOL (Qiagen). Subsequently, genomic DNAs were removed from 1 µg of the extracted RNAs by using ReverTraAce qPCR RT Master mix with gDNA remover (Toyobo Co., Ltd.) and reverse transcription was conducted to obtain cDNAs. The obtained cDNAs were 4-fold diluted with DDW, and with the diluted cDNAs as templates, RT-PCR was conducted using the following primers:
a GAPDH forward primer (SEQ ID NO: 12),
a GAPDH reverse primer (SEQ ID NO: 13),
an hSP3 forward primer (SEQ ID NO: 14), and
an hSP3 reverse primer (SEQ ID NO: 15). The RT-PCR products thus obtained were subjected to electrophoresis with 2% agarose gel (Nippon Gene Co., Ltd.). In addition, a single-cell clone (control clone) was established in the same manner as in the above (6) except that no donor DNA was transfected, and RT-PCR was conducted in the same manner.

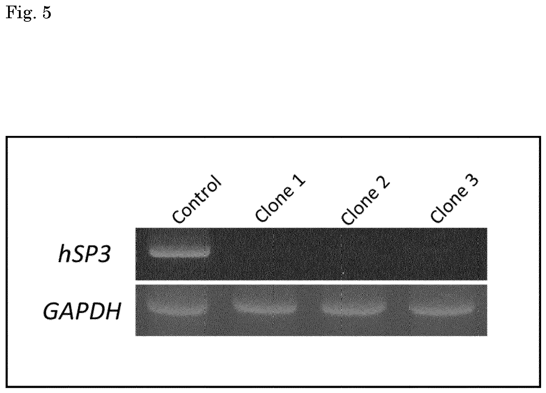

Fig. 5 shows electrophoresis pictures of the RT-PCR products obtained by conducting RT-PCR on the clones 1 to 3. Note that Fig. 5 also shows an electrophoresis picture of the RT-PCR product (Control) obtained by conducting RT-PCR on the control clone. As shown in Fig. 5, it was confirmed that the expression (transcription) of hSP3 was suppressed (no band of hSP3 was observed) in the clones (clones 1 to 3) in which the plasmid CRISPR1 and CRISPR2 as well as the donor DNA containing the methylated exogenous DNA were introduced, and that the donor DNA(methylated exogenous DNA) had been inserted into the target site of the hSP3 promoter.

### (8) Gene Knock-in Efficiency

In the same manner as in the above (7), cDNAs were obtained from the single-cell clones established in the above (6), RT-PCR was conducted with the cDNAs as templates, and electrophoresis was conducted on the RT-PCR products thus obtained. In addition, single-cell clones (control clones) were established in the same manner as in the above (6) except that no donor DNA was transfected, and RT-PCR was conducted in the same manner.

It was determined whether the donor DNA was inserted into the target site by confirming the band of hSP3 in each result of the electrophoresis. Among the 19 control clones (Controls 1 to 19) established without insertion of the donor DNA, there was no clone in which the expression (transcription) of hSP3 was suppressed (or the band of hSP3 was confirmed in all the control clones), and thus it was determined that the knock-in efficiency was 0%. On the other hand, among the 19 clones (clones 1 to 19) established by using the donor DNA, there were 6 clones in which the expression of hSP3 was suppressed, and thus it was determined that the knock-in efficiency was 31.6% (6/19×100). According to NPL 3, the knock-in efficiency of the method described in the document was approximately 20%. Hence, a higher knock-in efficiency than the conventional technique was achieved.

### (9) Bisulfite Sequencing

First, genomic DNAs were extracted from the single-cell clones established in the above (6) by using the Qiaamp DNA mini kit (Qiagen). Subsequently, bisulfite treatment was conducted on 200 ng of each genomic DNA thus extracted by using the EpiMark Bisulfite Conversion Kit (New England Biolabs).

With 50 ng of the genomic DNAs subjected to the bisulfite treatment as templates, PCR was conducted by using the following primers:
hSP3pro forward primer 2 (SEQ ID NO: 16), and
hSP3pro reverse primer 2 (SEQ ID NO: 17), and EpiTaq (New England Biolabs) to amplify the regions corresponding to the target site of the hSP3 promoter. Subsequently, the pUC19 plasmid (Nippon Gene Co., Ltd.) was cleaved by using the SmaI (New England Biolabs), and the PCR products thus obtained were cloned by using the Mightly ligation mix (Takara). The sequences thus cloned were determined by the Sanger Sequencing.

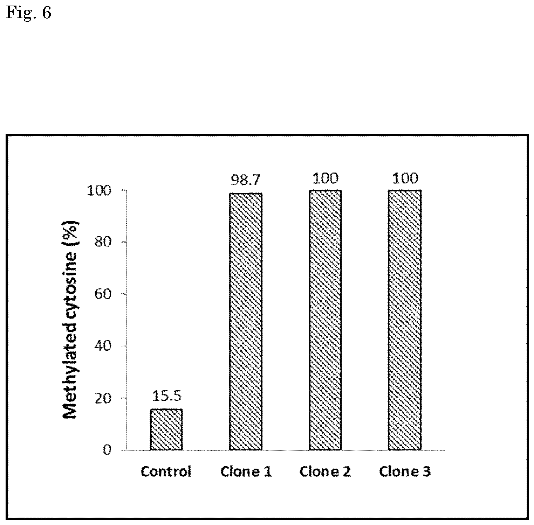

Fig. 6 shows the proportion of cytosine contained in each of the determined sequences (that is, the proportion of methylated cytosine. The non-methyl cytosine which was not methylated turned into uracil by the bisulfite treatment: methylated cytosine(%)=cytosine/(uracil+cytosine)×100). As shown in Fig. 6, in the clones (clones 1 to 3) in which the plasmid CRISPR1 and CRISPR2 as well as the donor DNA were introduced , it was confirmed that the proportion of methylated cytosine was almost 100% in the region corresponding to the target site of the hSP3 promoter.

As described above, it was confirmed that it is possible to knock in the methylated exogenous DNA(methylated hSP3 promoter) into 700 bp on the 3' side in the promoter of hSP3 highly efficiently by the above-described knock-in method.

### (Example 2)

### (1) Introduction of Methylation into B cell

First, 4 types of human B cells (JCRB cell bank, derived from Persons C, D, E, and F), purchased from the JCRB cell bank, were maintained and cultured in a medium (10% medium) obtained by adding 10% FBS (Hyclone blood serum, GE Healthcare) and 0.5% Penicillin-Streptomycin (Nacalai tesque, Inc.) to RPMI 1640 (Nacalai tesque, Inc.). Subsequently, the human B cells thus maintained and cultured were inoculated into a 24-well plate in 1×10⁵ cells/well.

Here, Persons C and E were B cells derived from humans (cancer patients) died of cancer (B-cell lymphoma), and Persons D and F were B cells derived from humans died of causes other than cancer. Persons C, D, E, and F did not have genes and mutations (driver genes and driver mutations) related to the conventional publicly-known malignant transformation.

On the next day following the inoculation, the combination of 150 ng/well of the plasmid CRISPR1 and 150 ng/well of the plasmid CRISPR2, which were constructed in Example 1, as well as 150 ng/well of the donor DNA was transfected by using polyethyleneimine (PEI, Polyplus-transfection SA). The medium was replaced with the above 10% medium on the next day following the transfection, and culturing was started in a medium to which 1 µg/mL of puromycin was added after 48 hours from the transfection. The medium was replaced with the above 10% medium after 48 hours from the start of adding puromycin, which was then maintained and cultured for 4 days.

In addition, as a control, the combination of 150 ng/well of the plasmid PX459-MSpCas9 (which did not contain the guide RNA), which was constructed in Example 1, and 150 ng/well of the donor DNA was maintained and cultured for 4 days in the same manner except that it was transfected by using polyethyleneimine (PEI, Polyplus-transfection SA), on the next day following the inoculation.

### (2) Cell Culturing

A Low Melt Agarose Gel (Bio-Rad Laboratories) was placed in a Bottom Layer 0.8% agarose gel 10% medium to obtain a 0.8% agarose gel solution, then the agarose gel was completely dissolved in a 80°C water bath, and dispensed into 10-cm dishes each in 5 mL. This was left standing at room temperature for 30 minutes to coagulate to prepare a bottom layer of an agar gel. Moreover, the Low Melt agarose gel was placed in a Cell-containing layer 0.3% agarose gel 10% medium to obtain a 0.3% agarose gel solution, and the agarose gel was completely dissolved in a 80°C water bath. This was left standing at room temperature to cool down to 37°C. The cells which had been maintained and cultured for 4 days in the above (1) were each suspended to be 1.25×10⁴ cells in every 5 mL, and dispensed on the Bottom layer each in 5 mL to obtain a Cell-containing layer. Once every 3 days, 10% medium of the 2.5 mL was added per 10-cm dish and they were maintained and cultured for 2 weeks.

### (3) Malignant Transformation Evaluation (Crystal Violet staining)

First, the cell-containing layer cultured in the above (2) was collected into a 50 mL tube, and an equivalent amount of 4% PFA (Wako) was added, which was left standing at room temperature for 20 minutes to fix the cells. Subsequently, the resultant was diluted with D-PBS (Nacalai tesque, Inc.) containing 10% FBS up to 50 mL, and was centrifuged at 1100 rpm for 10 minutes to precipitate the fixed cells, and the supernatant was removed.

Subsequently, Crystal Violet (Sigma) was diluted with 20% ethanol (Wako) to obtain 0.005% Crystal Violet. Then, 5 mL of the 0.005% Crystal Violet solution was added to the fixed cells thus precipitated, which were left standing at room temperature for 1 hour to be stained. Subsequently, the resultant was diluted with D-PBS containing 10% FBS up to 50 mL, and was centrifuged at 1100 rpm for 10 minutes to precipitate the fixed cells, and supernatant was removed. Subsequently, the stained cells were dissolved with 10 mL D-PBS, the entire amount of which was dispensed onto one 10 cm dish. This was left standing at room temperature for one night to be completely dried.

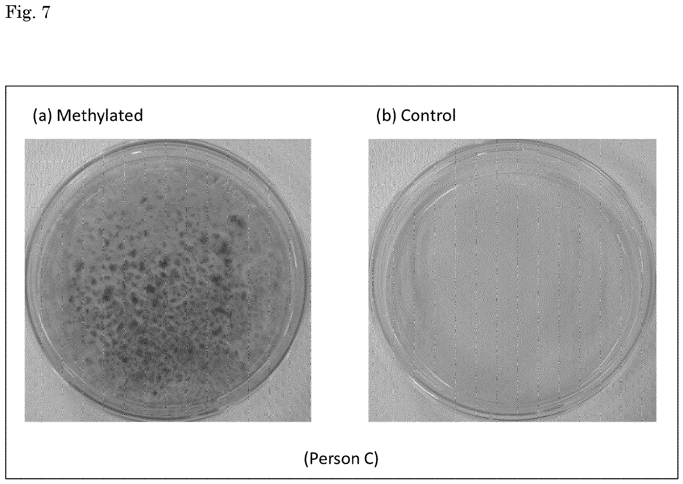

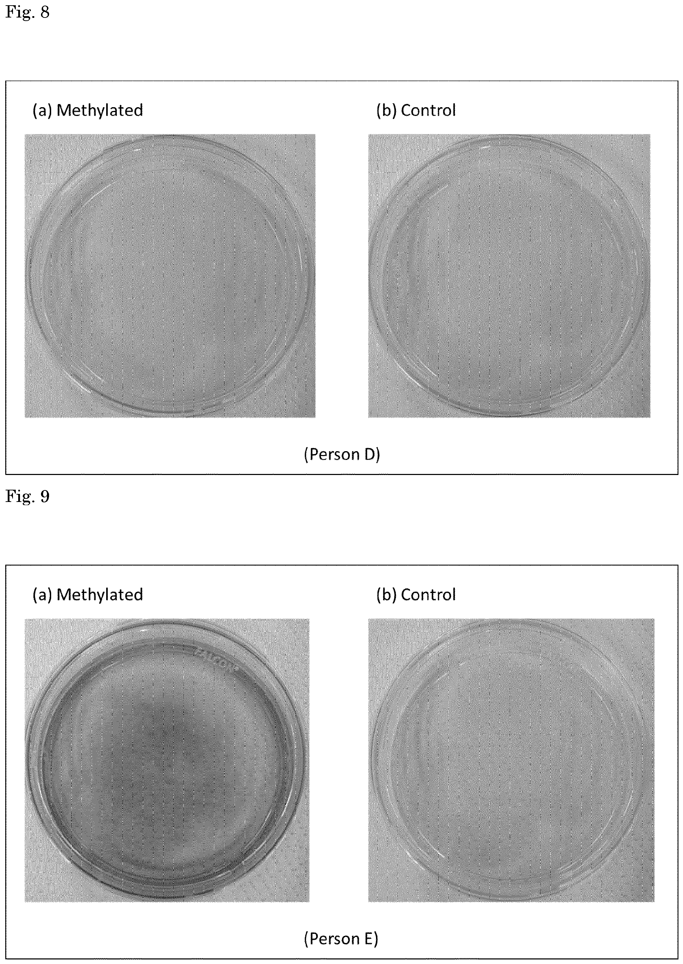

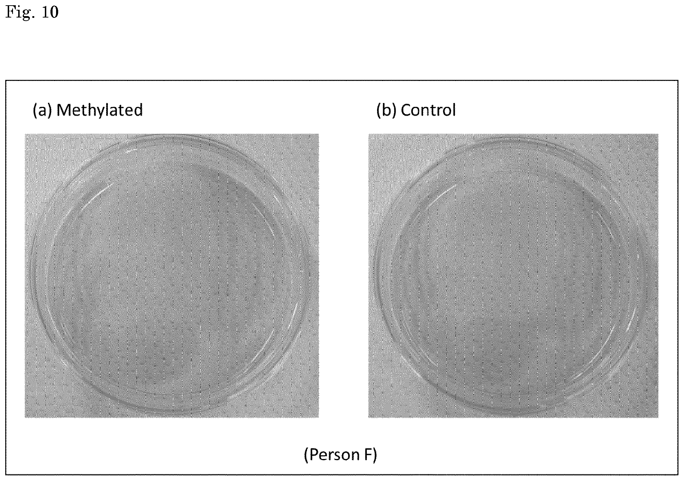

Fig. 7 (Person C), Fig. 8 (Person D), Fig. 9 (Person E), and Fig. 10 (Person F) show pictures showing external appearances of the 10 cm dishes after the drying, respectively. In Fig. 7 to Fig. 10, (a) each indicate results (Methylated) in the cells in which the combinations of the plasmid CRISPR1, the plasmid CRISPR2, and the donor DNA were transfected to introduce methylation, and (b) indicates result (Control) in the control cells in which the combination of the plasmid PX459-MSpCas9 (containing no guide RNA) and the donor DNA was transfected. In the case where B cells have been turned into cancer though malignant transformation, the cells are capable of surviving in the agar gel culture of the above (2), and thus take on blue due to the above-described Crystal Violet staining. As shown in Fig. 7 to Fig. 10, it was confirmed that the malignant transformation of a cell is specifically induced by introducing methylation into the promoter of the SP3 gene of B cells derived from a cancer patient.

### [Industrial Applicability]

As described above, the present invention makes it possible to provide a novel gene knock-in method, gene knock-in cell fabrication method, gene knock-in cell, and kit for use in these that are capable of inserting a long-chain exogenous DNA into a target site of a genomic DNA more efficiently than the conventional techniques.

In addition, since the present invention makes it possible to insert an exogenous DNA having a long-chain (for example, 700 bp or more) and/or an exogenous DNA modified outside the cell into a target site of a genomic DNA highly efficiently, it is possible to suppress the expression of a specific gene by inserting an exogenous DNA having 700 bp or more 100%-methylated base pairs into a promoter of the gene, for example.

Moreover, the present invention makes it possible to provide a novel malignant transformation risk evaluation method that is capable of directly evaluating malignant transformation risk of a cell by methylating a promoter of a genomic DNA of the cell, and a cancer cell production method that does not cause sequence abnormality of a gene due to methylation of a promoter of a genomic DNA of a cell.

Hence, the present invention also makes it possible to provide a method (gene evaluation method) that is capable of evaluating whether or not the gene under control of the promoter is a gene involved in malignant transformation, and in the case where the cell is a cell derived from a test subject and the promoter is a promoter of a gene related to specific cancer, a method (test subject evaluation method) that is capable of evaluating malignant transformation risk in the future (risk of development of the specific cancer) directly in the test subject without depending on statistical indicators.

### [Reference Signs List]

- 100:: target DNA region
- 111:: target site (target promoter site)
- 121:: first cleavage target region
- 122:: second cleavage target region
- 131:: first nucleotide sequence
- 132:: second nucleotide sequence
- 101:: first cleavage target portion
- 102:: second cleavage target portion
- 200:: donor DNA
- 112:: exogenous DNA (methylated promoter site)
- 223:: third cleavage target region
- 224:: fourth cleavage target region
- 225:: fifth cleavage target region
- 226:: sixth cleavage target region
- 231:: 5'-microhomology region
- 232:: 3'-microhomology region
- 203:: third cleavage target portion
- 204:: fourth cleavage target portion
- 205:: fifth cleavage target portion
- 206:: sixth cleavage target portion

### [Sequence Listing Free Text]

SEQ ID NO: 1
   <223> MSpCas9
SEQ ID NO: 2
   <223> MSpCas9 forward primer
SEQ ID NO: 3
   <223> MSpCas9 reverse primer
SEQ ID NO: 4
   <223> sgRNA1
SEQ ID NO: 5
   <223> sgRNA2
SEQ ID NO: 6
   <223> sgRNA3
SEQ ID NO: 7
   <223> sgRNA6
SEQ ID NO: 8
   <223> donor hSP3pro forward primer
SEQ ID NO: 9
   <223> donor hSP3pro reverse primer
SEQ ID NO: 10
   <223> hSP3pro forward primer
SEQ ID NO: 11
   <223> hSP3pro reverse primer
SEQ ID NO: 12
   <223> GAPDH forward primer
SEQ ID NO: 13
   <223> GAPDH reverse primer
SEQ ID NO: 14
   <223> hSP3 forward primer
SEQ ID NO: 15
   <223> hSP3 reverse primer
SEQ ID NO: 16
   <223> hSP3pro forward primer 2
SEQ ID NO: 17
   <223> hSP3pro reverse primer 2

## Claims

1. A gene knock-in method for knocking in an exogenous DNA into a target site of a genomic DNA, the method comprising:
a step of introducing a donor DNA that contains the exogenous DNA and a CRISPR-Cas9 system that includes a Cas9 protein and a guide RNA for the Cas9 protein as constituent elements into a cell, wherein
the donor DNA contains in order from a 5' side: a 5'-microhomology region formed of a first' nucleotide sequence that is capable of being joined to a first nucleotide sequence on a 5' side of the target site of the genomic DNA by microhomology-mediated end joining; the exogenous DNA; and a 3'-microhomology region formed of a second' nucleotide sequence that is capable of being joined to a second nucleotide sequence on a 3' side of the target site of the genomic DNA by microhomology-mediated end joining,
the CRISPR-Cas9 system
cleaves, in the genomic DNA, a first cleavage target region between the target site and the first nucleotide sequence and a second cleavage target region between the target site and the second nucleotide sequence, and
cleaves, in the donor DNA, a third cleavage target region on the 5' side of the 5'-microhomology region, a fourth cleavage target region between the exogenous DNA and the 5'-microhomology region, a fifth cleavage target region between the exogenous DNA and the 3'-microhomology region, and a sixth cleavage target region on the 3' side of the 3'-microhomology region, and
the exogenous DNA is inserted between the first nucleotide sequence and the second nucleotide sequence of the genomic DNA.

2. The gene knock-in method according to claim 1, wherein
the first cleavage target region and the second cleavage target region of the genomic DNA as well as the third cleavage target region, the fourth cleavage target region, the fifth cleavage target region, and the sixth cleavage target region of the donor DNA respectively contain a first guide RNA-target sequence, a second guide RNA-target sequence, a third guide RNA-target sequence, a fourth guide RNA-target sequence, a fifth guide RNA-target sequence, and a sixth guide RNA-target sequence that are recognized by the guide RNA.

3. The gene knock-in method according to claim 1 or 2, wherein
the Cas9 protein is a protein that contains point mutations of N692A, M694A, Q695A, and H698A in a wild-type Streptococcus pyogenes Cas9 (SpCas9) protein.

4. The gene knock-in method according to any one of claims 1 to 3, wherein
a length of the exogenous DNA of the donor DNA is 700 bp or more.

5. The gene knock-in method according to any one of claims 1 to 4, wherein
the exogenous DNA of the donor DNA is methylated.

6. The gene knock-in method according to claim 5, wherein
the target site of the genomic DNA is contained in a promoter, and
the exogenous DNA is the target site that is methylated.

7. A gene knock-in cell fabrication method for fabricating a cell in which the exogenous DNA is inserted into a target site of a genomic DNA by using the gene knock-in method according to any one of claims 1 to 6.

8. A gene knock-in cell in which an exogenous DNA containing 700 bp or more methylated base pairs is introduced.

9. A malignant transformation risk evaluation method for evaluating malignant transformation risk of a target cell, the method comprising:
a methylation step of methylating a target promoter site contained in a promoter of a genomic DNA in the target cell;
a detection step of detecting malignant transformation of the target cell; and
a cell evaluation step of evaluating the malignant transformation risk of the cell by using the malignant transformation of the target cell as an indicator.

10. The malignant transformation risk evaluation method according to claim 9, wherein
a length of the target promoter site is 700 bp or more.

11. The malignant transformation risk evaluation method according to claim 9 or 10, wherein
the methylation step is a step of knocking in an exogenous methylated promoter site into the target promoter site of a genomic DNA in the target cell by using the gene knock-in method according to any one of claims 1 to 6,
the target site is the target promoter site, and
the exogenous DNA is the methylated promoter site.

12. The malignant transformation risk evaluation method according to any one of claims 9 to 11, further comprising:
a gene evaluation step of evaluating malignant transformation capability of a gene controlled by a promoter containing the target promoter site by using the malignant transformation of the target cell as an indicator.

13. The malignant transformation risk evaluation method according to any one of claims 9 to 11, wherein
the target cell is a cell derived from a test subject, and
the promoter containing the target promoter site is a promoter of at least one gene selected from the group consisting of SP3, CDKN2A, p53, p21, and TERT1,
the method further comprising:
a test subject evaluation step of evaluating malignant transformation risk of the test subject by using the malignant transformation of the target cell as an indicator.

14. The malignant transformation risk evaluation method according to claim 13, wherein
the target cell is at least one selected from the group consisting of a B cell, a liver cell, a gastric epithelial cell, and a mucosal cell of pancreatic duct.

15. A cancer cell production method comprising:
a step of obtaining a cancer cell by methylating a target promoter site contained in a promoter of a genomic DNA in a cell.

16. The cancer cell production method according to claim 15, wherein
a length of the target promoter site is 700 bp or more.

17. The cancer cell production method according to claim 15 or 16, wherein
the methylation is methylation to knock in the exogenous methylated promoter site into the target promoter site of a genomic DNA in the cell by using the gene knock-in method according to any one of claims 1 to 6,
the target site is the target promoter site, and
the exogenous DNA is the methylated promoter site.

18. A gene knock-in kit for use in the gene knock-in method according to any one of claims 1 to 6, the kit comprising:
at least one selected from the group consisting of
(a) a Cas9 protein cleaving a first cleavage target region and a second cleavage target region of the genomic DNA as well as a third cleavage target region, a fourth cleavage target region, a fifth cleavage target region, and a sixth cleavage target region of the donor DNA, a polynucleotide encoding the Cas9 protein, or a vector expressing the Cas9 protein; and
(b) a guide RNA of the Cas9 protein, a polynucleotide encoding the guide RNA, or a vector expressing the guide RNA.

19. The gene knock-in kit according to claim 18, wherein
the guide RNA contains a first guide RNA, a second guide RNA, a third guide RNA, a fourth guide RNA, a fifth guide RNA, and a sixth guide RNA that respectively recognize a first guide RNA-target sequence and a second guide RNA-target sequence of the genomic DNA as well as a third guide RNA-target sequence, a fourth guide RNA-target sequence, a fifth guide RNA-target sequence, and a sixth guide RNA-target sequence of the donor DNA.

20. The gene knock-in kit according to claim 18 or 19, wherein
the Cas9 protein is a protein containing point mutations of N692A, M694A, Q695A, and H698A in a wild-type Streptococcus pyogenes Cas9 (SpCas9) protein.
